# EUROPEAN PATENT SPECIFICATION

(11) **EP 1 891 228 B1**
(45) Date of publication and mention of the grant of the patent: **14.09.2016**
(21) Application number: 06763480.8
(22) Date of filing: 02.06.2006
(51) Int. Cl.: C12Q 1/44, C12N 9/16, A61P 17/06, A61P 35/00

(54) **HDAC REGULATION ASSAYS, COMPOUNDS AND THERAPEUTIC COMPOSITIONS**
HDAC-REGULATIONSTESTS, VERBINDUNGEN UND THERAPEUTISCHE ZUSAMMENSETZUNGEN
ESSAIS DE REGULATION DE LA HDAC, COMPOSES ET COMPOSITIONS THERAPEUTIQUES

(30) Priority: 06.06.2005 EP 05104907; 28.10.2005 EP 05110148
(43) Date of publication of application: 27.02.2008
(73) Proprietor: Janssen Pharmaceutica NV, 2340 Beerse (BE)
(72) Inventor: DE SCHEPPER, Stefanie Helena, B-1741 Wambeek (BE); ARTS, Janine, B-2340 Beerse (BE); VIALARD, Jorge Eduardo, B-2340 Beerse (BE); ANDRIES, Luc Joseph, B-9100 Sint-niklaas (BE)
(74) Representative: Quaghebeur, Luc
(86) International application number: PCT/EP2006/062859
(87) International publication number: WO 2006/131496

(56) References cited:
- EP-A- 1 400 806
- WO-A2-2005/005475
- DATABASE UniProt [Online] 1 November 1997 (1997-11-01), "Histone deacetylase 1 (HD1)" XP002407974 retrieved from EBI Database accession no. Q13547

## Description

This invention relates to the field of assays for compounds that interact with the binding of a PTB-containing protein, i.e. APPL (Adaptor protein containing PH domain, PTB domain and Leucine zipper motif) with histone deacetylase, in particular HDAC1. Compounds identified using said assays are useful in inhibiting HDAC activity and as a medicine, in particular in the manufacture of a medicament to inhibit proliferative conditions, such as cancer and psoriasis.

### BACKGROUND OF THE INVENTION

Nuclear histones are known as integral and dynamic components of the machinery responsible for regulating gene transcription and other DNA-templated processes such as replication, repair, recombination, and chromosome segregation. They are the subject of post-translational modifications including acetylation, phosphorylation, methylation, ubiquitination, and ADP-ribosylation.

Histone deacetylase(s), herein referred to as "HDACs", are enzymes that catalyze the removal of the acetyl modification on lysine residues of proteins, including the core nucleosomal histones H2A, H2B, H3 and H4. Together with histone acetyltransferase(s), herein referred to as "HATs", HDACs regulate the level of acetylation of the histones. The balance of acetylation of nucleosomal histones plays an important role in transcription of many genes. Hypoacetylation of histones is associated with condensed chromatin structure resulting in the repression of gene transcription, whereas acetylated histones are associated with a more open chromatin structure and activation of transcription.

Eleven structurally related HDACs have been described and fall into two classes. Class I HDACs consist of HDAC 1, 2, 3, 8 and 11 whereas class II HDACs consist of HDAC 4, 5, 6, 7, 9 and 10. Members of a third class of HDACs are structurally unrelated to the class I and class II HDACs. Class I/II HDACs operate by zinc-dependent mechanisms, whereas class III HDACs are NAD-dependent.

In addition to histones, other proteins have also been the substrate for acetylation, in particular transcriptionfactors such as p53, GATA-1 and E2F; nuclear receptors such as the glucocorticoid receptor, the thyroid receptors, the estrogen receptors; and cell-cycle regulating proteins such as pRb. Acetylation of proteins has been linked with protein stabilization, such as p53 stabilization, recruitment of cofactors and increased DNA binding. p53 is a tumour suppressor that can induce cell cycle arrest or apoptosis in response to a variety of stress signals, such as DNA damage. The main target for p53-induced cell cycle arrest seems to be the p21 gene. Next to its activation by p53, p21 has been identified by virtue of its association with cyclin/cyclin-dependent kinase complexes resulting in cell cycle arrest at both G1 and G2 phases, its up-regulation during senescence, and its interaction with the proliferating cell nuclear antigen.

As already mentioned hereinbefore, histone acetylation and deacetylation play a key role in regulating gene transcription. The acetylation status of lysine residues on the N-terminal histone tails is tightly controlled by the dynamic equilibrium between competing activities of histone acetyltransferases (HATs) and deacetylases (HDACs). The HDAC-mediated decreased histone acetylation status is associated with transcriptional repression. Disruption of HAT or HDAC activity can be associated with the development of cancer. Genes encoding for HAT enzymes are mutated, amplified, translocated or overexpressed in tumors from hematological and epithelial origin, e.g. acute myeloid leukemia (AML), colorectal cancer, gastric cancer, and breast cancer. Deregulated and constant HDAC recruitment in conjunction with oncogenic transcription factors to the chromatin is observed in specific forms of leukaemia and lymphoma, such as acute promyelocytic leukemia (APL), non-Hodgkin's lymphoma and AML M2 subtype.

The study of inhibitors of HDACs indicates that they play an important role in cell cycle arrest, cellular differentiation, apoptosis and reversal of transformed phenotypes. Several HDAC inhibitors are currently in phase II of clinical development [reviewed in Arts et al.¹]) HDAC inhibition induces cell cycle arrest, cell differentiation and apoptosis.

The inhibitor Trichostatin A (TSA), for example, causes cell cycle arrest at both G1 and G2 phases, reverts the transformed phenotype of different cell lines, and induces differentiation of Friend leukemia cells and others. TSA (and suberoylanilide hydroxamic acid SAHA) have been reported to inhibit cell growth, induce terminal differentiation, and prevent the formation of tumours in mice (Finnin et al., Nature, 401: 188-193, 1999).

Trichostatin A has also been reported to be useful in the treatment of fibrosis, e.g. liver fibrosis and liver chirrhosis. (Geerts et al., European Patent Application EP 0 827 742, published 11 March, 1998).

HDAC inhibitors can have indirect activities such as augmentation of the host immune respons and inhibiton of tumor angiogenesis and thus can suppress the growth of primary tumors and impede metastasis (Mai et al., Medicinal Research Reviews, 25: 261-309).

In addition, inhibitors of HDACs have also been reported to induce p21 gene expression. The transcriptional activation of the p21 gene by these inhibitors is promoted by chromatin remodelling, following acetylation of histones H3 and H4 in the p21 promotor region. This activation of p21 occurs in a p53-independent fashion and thus HDAC inhibitors are operative in cells with mutated p53 genes, a hallmark of numerous tumours.

So has it been demonstrated that disruption of HDAC 1 in mouse embryonic stem cells results in severely impaired cell proliferation and embryonic lethality *in vivo* (Lagger et al.²). This correlated with an increase in H3 and H4 acetylation and increased expression of the cyclin dependent kinase inhibitors p21*^{waf1,cip1}* and p27. Moreover, knockdown of HDAC 1 causes inhibition of cell proliferation and morphological changes indicative of cell differentiation.

HDAC 1 exerts its function as a component of at least 3 different multi-protein complexes: the Sin3 complex, the NuRD/Mi2 complex and the CoREST complex [reviewed in Sengupta and Seto³]. Residing in these complexes is crucial for HDAC1's enzymatic activity. Besides complex formation, HDAC activity is regulated through posttranslational modifications. Phosphorylation of HDAC1 on serine 421 and serine 423 by CK2 stabilizes complex formation with RbAp48, MTA-2, Sin3 and CoREST, thereby enhancing HDAC1 enzymatic activity⁴. HDAC1 can also be ubiquitinated and sumoylated on several C-terminal lysine-residues^{5,6}. SUMO1 modifications enhance the transcriptional repression by HDAC 1 without effecting complex formation.

In view of the above, HDAC inhibitors can have great potential in the treatment of cell proliferative diseases or conditions, including tumours with mutated p53 genes. The pharmacophore of the current HDAC inhibitors is focussed around the zinc-containing active site of the HDACs. In order to expand the chemical space for HDAC inhibition, it would be of interest to identify other binding partners that may influence the function and regulation of HDAC proteins, in particular regulators of HDAC enzymatic activity.

It is an object of the present invention to provide the identification of a novel HDAC co-factor, i.e. APPL (adaptor protein containing PH domain, PTB domain, and Leucine sipper motif) that enhances HDAC activity upon binding and the consequent use thereof in identifying HDAC inhibitors , i.e compounds that affect the interaction between HDAC and APPL. These and other aspects of the invention are described herein in more detail.

### SUMMARY OF THE INVENTION

The present invention provides assays that make use of the interaction of APPL or a fragment thereof, with a HDAC enzyme, in particular HDAC1 or a fragment thereof capable to interact with APPL or said APPL fragment. APPL, also known as DIP13α, stands for "adaptor protein containing a PH domain, a PTB domain and Leucine zipper motif"⁷⁻¹⁰ and has been identified as a novel direct binding partner of HDAC in eukaryotic cells and a key factor in the regulation of its enzymatic activity. The assays are useful to identify whether a test compound can alter the interaction of APPL with HDAC. The assays are also useful to determine whether the test compound is an agonist or antagonist of HDACs. The above assays can be performed in a variety of formats including competitive, non-competitive and comparative assays in which the interaction of APPL (SEQ ID NO:2) or related peptides with HDAC is assessed as a positive or negative control or compared to the results obtained with the test compound.

In another aspect the present invention relates to the isolated and purified polypeptide and polynucleotide molecules encoding the HDAC binding fragment of APPL consisting of the amino acids 500-635 of APPL (SEQ ID NO:3) as well as homologs thereof and the diagnostic and therapeutic use thereof.

In a further aspect the present invention relates to the identification of the APPL binding region, hereinafter also refered to as APPL binding site or APPL binding site, in the HDAC domain and to the isolated and purified polypeptide and polynucleotide molecules encoding said APPL binding region consisting of amino acids 51-84 of HDAC1 (SEQ ID NO:7) as well as homologs thereof and the use of said APPL binding region in the assays according to the invention.

In a further embodiment the present invention relates identifying compounds in the assays provided by the invention, and the therapeutic use thereof to inhibit proliferative conditions, such as cancer and psoriasis. This invention provides a method for inhibiting the abnormal growth of cells, including transformed cells, by administering an effective amount of a compound of the invention. Abnormal growth of cells refers to cell growth independent of normal regulatory mechanisms (e.g. loss of contact inhibition). This includes the inhibition of tumour growth both directly by causing growth arrest, terminal differentiation and/or apoptosis of cancer cells, and indirectly, by inhibiting neovascularization of tumours.

Further a method for isolating HDACs from a cellular fraction containing the same is disclosed, comprising contacting the cellular fraction with APPL or a HDAC binding fragment thereof immobilized to a solute substrate and eluting HDACs therefrom.

### BRIEF DESCRIPTION OF THE DRAWING

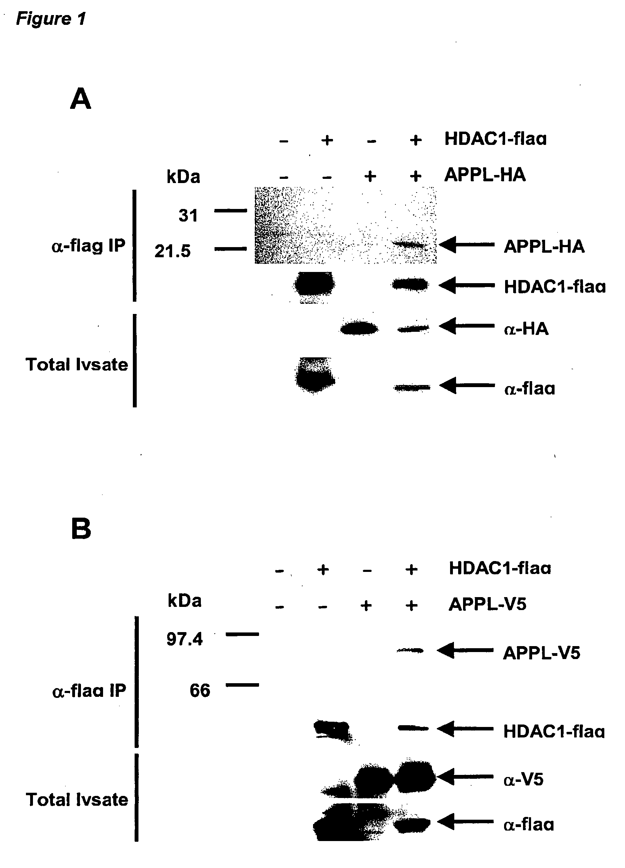

Figure 1 HDAC1 coimmunoprecipitates the APPL-PTB domain and the full length APPL.
   HEK293 cells were transfected using Lipofectamine Plus reagent with the indicated combinations of HDAC 1-flag and HA-APPL-PTB (panel **A**) or V5-APPL (panel **B**) for 24 hours. Thereafter, total cell lysates were prepared by lysis in a low stringency buffer, containing 50 mM Tris-HCL (pH 7.5), 120 mM NaCl, 5 mM EDTA, and 0.5% Nonidet P-40. HDAC1 immunoprecipitated proteins were separated by SDS-PAGE and the coimmunoprecipitation of HA-APPL-PTB (**A**) or V5-APPL (**B**) was monitored by Western blotting. As a control, the total amount of HDAC-flag protein that was immunoprecipitated was revealed as well as the HDAC1-flag, HA-APPL-PTB and V5-APPL overexpression levels in the total lysates.

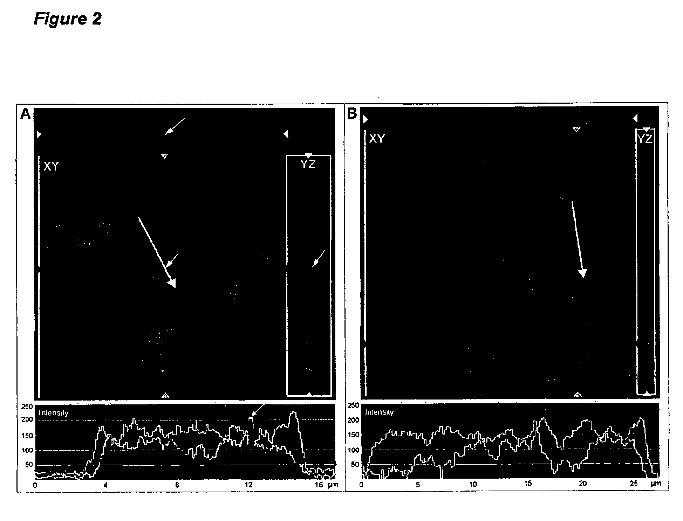

Figure 2 APPL and HDAC1 colocalization in MDA-MB-231 and A2780 cells. MDA-MB-231 and A2780 cells were seeded in 8 well culture chamber slides and fixed with 4% paraformaldehyde in Millonig buffer (panel A and B) or with cold methanol (panel C and D). Slides were incubated with HDAC1 and APPL specific Antibodies (Upstate Biotechnology) and APPL (custom manifactured by Eurogentech), and than incubated with Alexa488 conjugated anti-mouse for HDAC1 (green) and Cy3 conjugated anti-rabbit for APPL (red). DNA was stained with Hoechst (blue). Panel A and B: Composite images illustrating 3D-distribution of HDAC1 and APPL during interphase in nuclei of MDA-MB-231 (panel A) and A2780 (panel B) cells were composed from Z-stack series of optical sections using a motorized Axioplan 2 (Zeiss) microscope equipped with an Apotome and a Axiocam HR. The outer panels show the XZ (red rectangle) and YZ (blue rectangle) dimensions through the Z-stack. The white triangles in the outer panels indicate the location of the XY image in the Z-stack. The pixel intensity graph represent the pixel values of the 3 channels (blue=Hoechst; green=HDAC1, red=APPL) along the yellow arrow in the XY image. The white arrows in **A** mark an area with high pixel values for both HDAC1 and APPL.

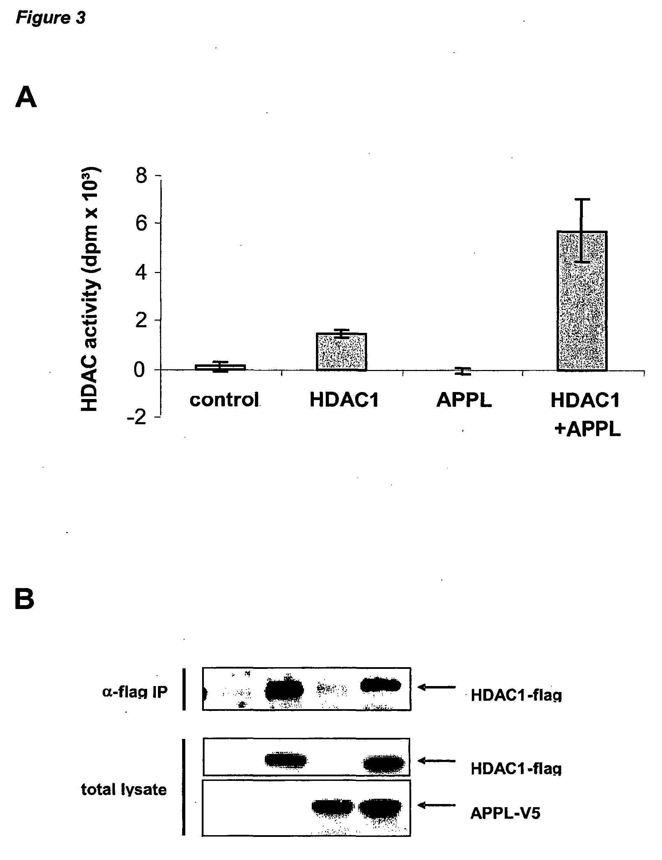

Figure 3 APPL overexpression increases HDAC1 enzymatic activity in HEK293 cells. HEK293 cells were transfected with the indicated combinations of HDAC1-flag and V5-APPL for 48 hours. Panel **A**: HDAC1 activity was measured by incubating immunoprecipitated HDAC1 complexes with a [³H]acetyl-labeled fragment of histone H4 peptide ([biotin-(6-aminohexanoic)Gly-Ala-(acetyl[³H])Lys-Arg-His-Arg-Lys-Val-NH₂], Amersham Pharmacia Biotech). Released [³H]acetic acid was extracted with ethyl acetate and quantified by scintillation counting. HDAC1 activity results are presented as mean ± standard deviation of 3 independent experiments on a single lysate. Panel **B**: Equal amounts of HDAC1 were immunoprecipitated as indicated by Western blot analysis. The amount of HDAC1-flag and V5-APPL protein that was overexpressed was revealed in the total cell lysates by Western blotting.

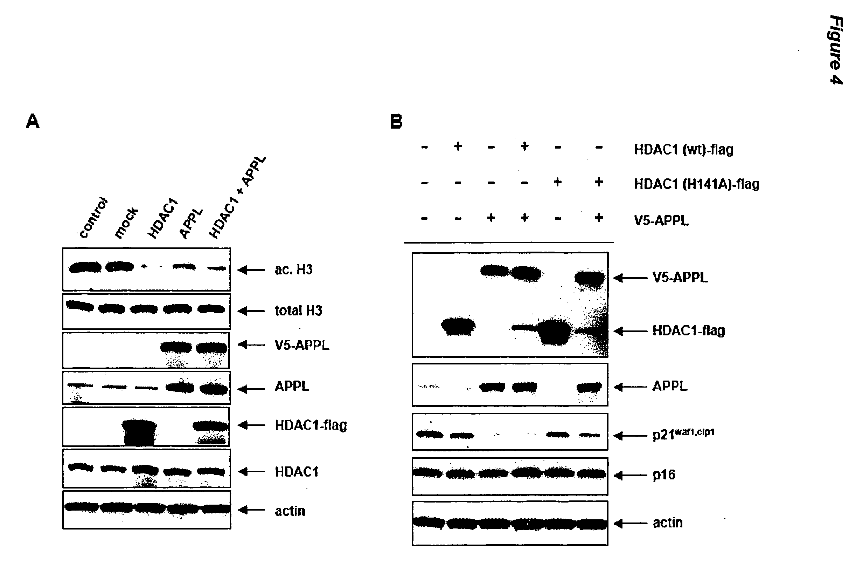

Figure 4 Overexpression of APPL decreases H3 acetylation and p21*^{waf1,cip1}* protein expression.
   HEK293 cells were transfected with the indicated combinations of HDAC1-flag and V5-APPL for 48 hours. Panel A: the transfected cell population was enriched using the MACSelect-transfected Cell Selection System (Miltenyi Biotec). Panel A and B: Total cell lysates were prepared in RIPA buffer and proteins were separated by SDS-PAGE. Protein expression was analysed by Western blotting using specific antibodies for acetylated H3 (Upstate Biotechnology), total H3 (abcam), V5-APPL (Invitrogen), APPL (Eurogentech), HDAC1-flag (Sigma), p21*^{waf1,cip1}* (Transduction Laboratories), and p16 (BD Pharmingen). Actin protein levels (Oncogene) were revealed as a control for equal loading. Protein-antibody complexes were visualized by chemiluminescence (Pierce Chemical Co.) and fluorescence (Odyssey) according to manufacturer's instructions.

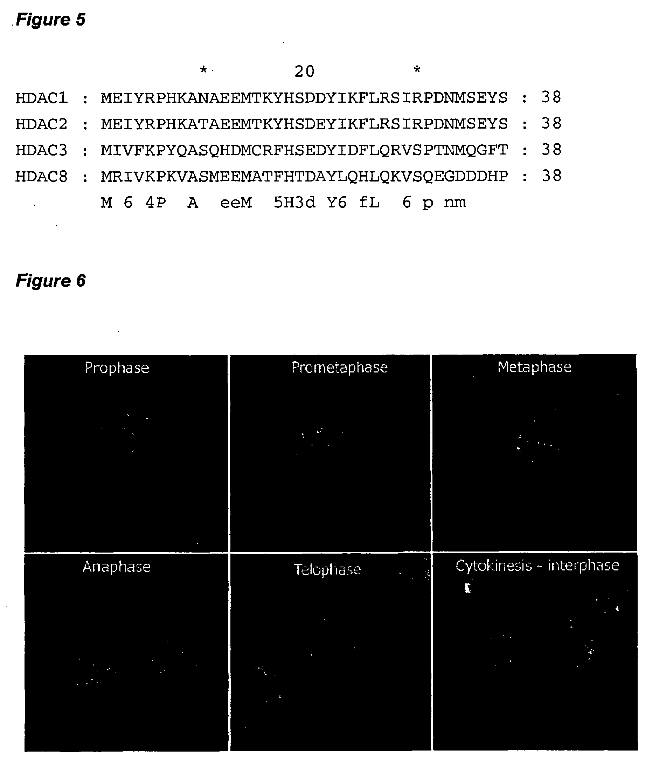

Figure 5 Alignment of all APPL-PTB binding partners at the PTB binding domain. Sequence names are provided as; UniProt Accession Number_HUGO gene symbol_species. The bottom line provides a consensus sequence wherein 3 represents the carbohydrates Serine (S) or Threonine (T); 4 represents the basic amino acid residues Lysine (K) or Arginine (R); 5 represents the aromatic amino acid residues Phenylalanine (F), Tyrosine (Y) or Tryptophan (W); and 6 represents the hydrophobic amino acid residues Leucine (L), Isoleucine (I), Valine (V) or Methionine (M).
Figure 6 Apotome images of MDA-MB-231 cells at different mitotic stages. MDA-MB-231 cells were seeded in 8 well culture chamber slides and fixed with cold methanol. Slides where incubated with HDAC1- (Upstate Biotechnology) and APPL- (cutom manufactured by Eurogentech) specific antibodies and than incubated with Alexa488 conjugated anti-mouse for HDAC1 (green) and Cy3 conjugated anti-rabbit for APPL (red). DNA was stained with Hoechst (blue).

### DETAILED DESCRIPTION

As used herein, "APPL" refers to the adaptor protein containing a PH domain, a PTB domain and Leucine zipper motif, also known as DIP13α having the amino acid sequence SEQ ID NO: **2** or APPL-related proteins wherein said related proteins are derived from the aforementioned sequence by way of substitution, deletion and/or addition of one or several amino acids of the amino acid sequence encoding APPL and wherein said APPL-related proteins have at least 70, 80, 85, 90, 95, 97, 98 or 99% identity to SEQ ID NO:**2** and are capable of binding to the HDAC proteins according to the invention. In another embodiment the present invention provides fragments of the APPL proteins wherein said fragments comprise at least 100, 150, 200, 250 or 300 amino acids that are contiguous in the parent protein and wherein said fragments are capable of binding HDAC or the APPL binding fragment thereof. In a particular embodiment said fragment consists of the HDAC binding fragment of APPL (SEQ ID NO:**3**) as well as homologs thereof wherein said homologs have at least 70, 80, 85, 90, 95, 97, 98 or 99% identity to SEQ ID NO:**3** and are capable to bind to the HDAC proteins, in particular to HDAC 1. The APPL fragments as defined hereinbefore are meant to be within the definition of APPL-related proteins as used throughout the present text.

As used hereinbefore "HDACs" are enzymes that catalyze the removal of the acetyl modification on lysine residues of proteins, including the core nucleosomal histones H2A, H2B, H3 and H4. As used in the present invention, these enzymes consist of the HDAC proteins 1 to 11, in particular HDAC 1 having the amino acid sequence SEQ ID NO:**5** or homologs thereof wherein said homologs have at least 70, 80, 85, 90, 95, 97, 98 or 99% identity to SEQ ID NO:**5** and are capable to bind to APPL or the HDAC binding fragment of APPL. It is also an object of the present invention to provide fragments of said HDAC proteins wherein said fragments comprise at least 20 amino acids that are contiguous in the parent protein, but may desirably contain at least 40, 60, 80, 100, 150, 200, 250, 300, or 350 amino acids that are contiguous in the parent protein and wherein said fragments are capable of binding APPL or the HDAC binding fragment of APPL. In a particular embodiment said fragment comprises the histone deacetylase domain (SEQ ID NO:**6**) or homologs thereof wherein said homologs have at least 70, 80, 85, 90, 95, 97, 98 or 99% identity to SEQ ID NO:**6**. In a more particular embodiment said fragments comprise the APPL binding region consisting of the amino acids 51-84 of HDAC1 (SEQ ID NO:**7**) or homologs thereof wherein said homologs have at least 70, 80, 85, 90, 95, 97, 98 or 99% sequence identity to SEQ ID No:7. The homologs of the APPL binding region may be characterised in having a Methionine (M) at positions 1 and 14, a hydrophobic amino acid at position 3; a Proline (P) at positions 6 and 31; an Alanine (A) at position 9; a Glutamate (E) at positions 12 and 13, an aromatic amino acid at position 19 and a Histidine (H) at position 18 when compared to SEQ ID NO:7. As used herein an aromatic amino acid is selected from the group consisting of Phenylalanine (F), Tyrosine (Y), Tryptophan (W) or Histidine (H) and a hydrophobic amino acid is selected from the group consisting of Isoleucine (I), Leucine (L), Valine (V), Cysteine (C), Alanine (A), Glycine (G), Methionine (N), Phenylalanine (F), Tyrosine (Y), Tryptophan (W), Histidine (H), Threonine (T) or Proline (P). In an even further embodiment the HDAC binding fragment of APPL consists of SEQ ID NO:**6** or SEQ ID NO:**7**.

Methods for comparing the identity and similarity of two or more sequences are well known in the art. Thus for instance, programs available in the Winconsin Sequence Analysis Package, version 9.1 (Devreux J. et al, Nucleic Acid Res., 12, 387-395, 1984), for example the programs BESTFIT and GAP, may be used to determine the % identity between two polynucleotides and the % identity and the % similarity between two peptide or polypeptide sequences. BESTFIT uses the "local homology" algorithm of Smith and Waterman (J. Mol. Biol., 147, 195-197, 1981) and finds the best single region of similarity between two sequences. BESTFIT is more suited to compare two polynucleotide or two peptide or polypeptide sequences that are dissimilar in length, the program assuming that the shorter sequence represents a portion of the longer. In comparison, GAP aligns two sequences, finding a "maximum similarity", according to the algorithm of Needleman and Wunsch (J.Mol.Biol., 48, 443-453, 1970). GAP is more suited to compare sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned. Other programs for determining identity and/or similarity between sequences are also known in the art, for instance the BLAST family of programs (Altschul S F et al, Nucleic Acids Res., 25:3389-3402, 1997).

As used herein, a "compound" is an organic or inorganic assembly of atoms of any size, and includes small molecules (less than about 2500 Daltons) or larger molecules, e.g. peptides, polypeptides, whole proteins and polynucleotides.

As used herein, a "test" compound is a compound used in a test to assess whether said test compound may be an agonist or antagonist of the HDAC enzyme. Whether or not the test compound is an actual agonist or antagonist of a HDAC enzyme is determined in an assay according to the invention.

As used herein, an "agonist" is a compound that interacts with and activates a HDAC enzyme. An activated HDAC enzyme will catalyze the removal of acetyl from acetylated lysine residues of proteins and can for example be assessed by measuring the release of acetic acid from a detectably labeled acetyl-histone peptide substrate.

As used herein, an "antagonist" is a compound that interacts with and inhibits or prevents the activation of a HDAC enzyme.

### Polynucleotides

Isolated and purified nucleic acid molecule which encodes APPL or a fragment thereof, wherein said nucleic acid molecule is either RNA, DNA, cDNA or genomic DNA, may be used for the invention of the interaction of APPL and the related peptides with the HDACs or fragments thereof.

Isolated and purified nucleic acid molecule encoding HDAC or fragments thereof, wherein said nucleic acid molecule is either RNA, DNA, cDNA or genomic DNA, may be used for the invention of the interaction of APPL and the APPL-related proteins with said HDAC or HDAC fragments.

As used herein, "isolated" refers to the fact that the polynucleotides, proteins and polypeptides, or respective fragments thereof in question, have been removed from their *in vivo* environment so that they can be manipulated by the skilled artisan, such as but not limited to sequencing, restriction digestion, site-directed mutagenesis, and subcloning into expression vectors for a nucleic acid fragment as well as obtaining the protein or protein fragments in quantities that afford the opportunity to generate polyclonal antibodies, monoclonal antibodies, amino acid sequencing, and peptide digestion. In other words "isolated" indicates that a naturally occurring sequence has been removed from its normal cellular context. Thus, the sequence may be in a cell-free solution or placed in a different cellular environment or nucleic acid context. Therefore, the nucleic acids claimed herein can be present as heterologous material in whole cells or in cell lysates or in a partially, substantially or wholy purified form.

A polynucleotide is considered "purified" when it is purified away from environmental contaminants. Thus a polynucleotide isolated from cells is considered to be substantially purified when purified from cellular components by standard methods while a chemically synthesized nucleic acid sequence is considered to be substantially purified when purified from its chemical precursors. A "substantially pure" protein or nucleic acid will typically comprise at least 85% of a sample with greater percentages being preferred. One method for determining the purity of a protein or nucleic acid molecule, is by electrophoresing a preparation in a matrix such as polyacrylamide or agarose. Purity is evidenced by the appearance of a single band after staining. Other methods for assessing purity include chromatography, mass spectrometry and analytical centrifugation.

The term "fragments thereof' describes a piece, or sub-region of a nucleic acid molecule whose sequence is disclosed herein, such that said fragment comprises 15 or more nucleotides that are contiguous in the parent nucleic acid molecule, but may desirably contain at least 40, 50 or 100 nucleotides of the parent nucleic acid molecule. The term "fragments thereof" is intended to include "functional fragments" wherein the isolated fragment, piece or sub-region comprises a functionally distinct region such as an active site, a binding site or a phosphorylation site of a receptor. Functional fragments may be produced by cloning technology, or as the natural products of alternative splicing techniques. As used in connection to HDAC proteins, a functional fragment retains the enzymatic activity of said proteins, i.e. removal of the acetyl modification of lysine residues in proteins, including the core nucleosomal histones H2A, H2B, H3 and H4. Their activity is typically assessed using an detectably labeled acetylated histone as a substrate and measuring the release of the labeled acetyl group such as for example provided in Example 4 hereinafter. Functional fragments of HDAC proteins would minimally comprise the histone deacetylase domain of said proteins, in particular the histone deacetylase domain of HDAC1 having the amino acid sequence SEQ ID NO:6 and encoded by nucleic acids 109 to 1047 of SEQ ID No.4, or homologs thereof wherein said homologs have at least 70, 80, 85, 90, 95, 96, 97, 98, or 99% sequence identity to nucleic acids 109 to 1047 of SEQ ID No.4.

For the APPL protein used in the invention an isolated and purified nucleic acid molecule encoding APPL or a fragment thereof, comprising a member selected from a group consisting of:
(a) a nucleic acid molecule encoding APPL comprising the amino acid sequence of SEQ ID NO:**2**;
(b) a nucleic acid molecule comprising the nucleic acid sequence encoding APPL (SEQ ID NO:**1**);
(c) a nucleic acid molecule encoding the HDAC binding fragment of APPL comprising the nucleic acids 1554 to 1958 of SEQ ID NO:**1** as well as homologs thereof wherein said homologs have at least 70, 80, 85, 90, 95, 97, 98 or 99% identity to said fragment of SEQ ID NO:**3**;
(d) a nucleic acid molecule encoding the HDAC binding fragment of APPL, comprising the amino acid sequence SEQ ID NO:**3**;
(e) a nucleic acid molecule which is complementary to the polynucleotide of (a) to (d);
(f) a nucleic acid molecule comprising at least 15 sequential bases of the polynucleotide of (a) to (d);
(g) a nucleic acid molecule that hybridizes under stringent conditions to the polynucleotide molecule of (a) to (d); or
(h) a nucleic acid molecule encoding APPL comprising a nucleotide sequence which is degenerated as a result of the genetic code to a nucleotide sequence of a polynucleotide of any of (a) to (g),
may be useful.

For the APPL protein used in the invention an isolated and purified nucleic acid molecule encoding APPL or a fragment thereof, comprising a member selected from a group consisting of:
(a) a nucleic acid molecule encoding APPL having the amino acid sequence of SEQ ID NO:**2**;
(b) a nucleic acid molecule comrpising the isolated nucleic acid sequence encoding APPL (SEQ ID NO:1);
(c) a nucleic acid molecule encoding the HDAC binding fragment of APPL consisting of the nucleic acids 1554 to 1958 of SEQ ID NO:**1** as well as homologs thereof wherein said homologs have at least 70, 80, 85, 90, 95, 97, 98 or 99% identity to the nucleic acids 1554 to 1958 of SEQ ID NO:**1**;
(d) a nucleic acid molecule encoding the HDAC binding fragment of APPL, said HDAC binding fragment having the amino acid sequence SEQ ID NO:**3**;
(e) a nucleic acid molecule which is complementary to the polynucleotide of (a) to (d);
(f) a nucleic acid molecule comprising at least 15 sequential bases of the polynucleotide of (a) to (d);
(g) a nucleic acid molecule that hybridizes under stringent conditions to the polynucleotide molecule of (a) to (d); or
(h) a nucleic acid molecule encoding APPL comprising a nucleotide sequence which is degenerated as a result of the genetic code to a nucleotide sequence of a polynucleotide of any of (a) to (g)
may be useful.

The APPL encoding nucleic acid molecule consists of SEQ ID NO: 1, and the HDAC binding fragment of APPL encoding nucleic acid molecule consists of the nucleic acids 1554 to 1958 of SEQ ID NO:**1.**

For the HDAC protein used in the invention an isolated and purified nucleic acid molecule encoding HDAC or a fragment thereof, comprising a member selected from a group consisting of:
(a) a nucleic acid molecule encoding HDAC1 comprising the amino acid sequence of SEQ ID NO:**5**;
(b) a nucleic acid molecule comprising the nucleotide sequence of SEQ ID NO:**4**, encoding HDAC 1;
(c) a nucleic acid molecule encoding the histone deacetylase domain of HDAC 1 comprising the nucleic acids 109 to 1047 of SEQ ID NO:**4** as well as thereof wherein said homologs have at least 70, 80, 85, 90, 95, 97, 98 or 99% identity to said fragment of SEQ ID NO:**4**;
(d) a nucleic acid molecule encoding the histone deacetylase domain of HDAC, comprising the amino acid sequence SEQ ID NO:**6**;
(e) a nucleic acid molecule encoding the APPL binding region of HDAC1 comprising the nucleic acids 235 to 336 of SEQ ID NO:**4** as well as homologs thereof wherein said homologs have at least 70, 80, 85, 90, 95, 97, 98 or 99% identity to said fragment of SEQ ID NO:**4**;
(f) a nucleic acid molecule encoding the APPL binding region of HDAC, comprising the amino acid sequence SEQ ID NO:**7**;
(g) a nucleic acid molecule which is complementary to the polynucleotide of (a) to (f);
(h) a nucleic acid molecule comprising at least 15 sequential bases of the polynucleotide of (a) to (g);
(i) a nucleic acid molecule that hybridizes under stringent conditions to the polynucleotide molecule of (a) to (f); or
(j) a nucleic acid molecule encoding HDAC comprising a nucleotide sequence which is degenerated as a result of the genetic code to a nucleotide sequence of a polynucleotide of any of (a) to (i),
may be useful.

For the HDAC protein used in the invention an isolated and purified nucleic acid molecule encoding HDAC or a fragment thereof, comprising a member selected from a group consisting of:
(a) a nucleic acid molecule encoding HDAC1 having the amino acid sequence of SEQ ID NO:**5**;
(b) a nucleic acid molecule consisting of the nucleotide sequence of SEQ ID NO:**4**, encoding HDAC1;
(c) a nucleic acid molecule encoding the histone deacetylase domain of HDAC 1 consisting of the nucleic acids 109 to 1047 of SEQ ID NO:**4** as well as thereof wherein said homologs have at least 70, 80, 85, 90, 95, 97, 98 or 99% identity to said fragment of SEQ ID NO:**4**;
(d) a nucleic acid molecule encoding the histone deacetylase domain of HDAC, having the amino acid sequence SEQ ID NO:**6**;
(e) a nucleic acid molecule encoding the APPL binding region of HDAC 1 consisting of the nucleic acids 235 to 336 of SEQ ID NO:**4** as well as homologs thereof wherein said homologs have at least 70, 80, 85, 90, 95, 97, 98 or 99% identity to said fragment of SEQ ID NO:**4**;
(f) a nucleic acid molecule encoding the APPL binding region of HDAC, having the amino acid sequence SEQ ID NO:**7** or homologs thereof wherein said homologs have at least 70, 80, 85, 90, 95, 97, 98 or 99% identity to SEQ ID No:**7**;
(g) a nucleic acid molecule which is complementary to the polynucleotide of (a) to (f);
(h) a nucleic acid molecule comprising at least 15 sequential bases of the polynucleotide of (a) to (f);
(i) a nucleic acid molecule that hybridizes under stringent conditions to the polynucleotide molecule of (a) to (f); or
(j) a nucleic acid molecule encoding HDAC comprising a nucleotide sequence which is degenerated as a result of the genetic code to a nucleotide sequence of a polynucleotide of any of (a) to (i),
may be useful.

In a particular embodiment of the present invention the HDAC encoding nucleic acid molecule consists of SEQ ID NO: **4**, and the APPL binding region encoding nucleic acid molecule consists of nucleic acides 109 to 1047 of SEQ ID NO:**4**, more in particular the fragment thereof consisting of nucleic acids 235 to 336 of SEQ ID NO:**4**.

Those skilled in the art will recognize that owing to the degeneracy of the genetic code, numerous "silent" substitutions of nucleotide base pairs could be introduced into the sequence identified as SEQ ID NO:**1**, SEQ ID NO:**4** or the above identified fragments of said sequences, without altering the identity of the encoded amino acid(s) or protein products. All such substitutions are intended to be within the scope of the invention.

The terms "complementary" or "complementarity" as used herein refer to the capacity of purine and pyrimidine nucleotides to associate through hydrogen bonding to form double-stranded nucleic acid molecules. The following base pairs are related by complementarity: guanine and cytosine; adenine and thymine; and adenine and uracil. As used herein "complementary" means that the aforementioned relationship applies to substantially all base pairs comprising two single-stranded nucleic acid molecules over the entire length of said molecules. "Partially complementary" refers to the aforementioned relationship in which one of the two single-stranded nucleic acid molecules is shorter in length than the other such that a portion of one of the molecules remains single-stranded.

The term "hybridization" as used herein refers to a process in which a single-stranded nucleic acid molecule joints with a complementary strand through nucleotide base pairing.

The term "stringency" refers to hybridization conditions. High stringency conditions disfavor non-homologous base pairing. Low stringency conditions have the opposite effect. Stringency may be altered, for example, by temperature and salt concentration. "Stringent conditions" refers to an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1 x SSC at about 65°C. Further suitable hybridization conditions are described in the examples.

For the HDAC binding fragment within APPL and the APPL binding fragment within HDAC used in the invention an isolated and purified nucleic acid molecule selected from a group consisting of:
(a) a nucleic acid molecule encoding the HDAC binding fragment of APPL consisting of the nucleic acids 1554 to 1958 of SEQ ID NO:**1** as well as homologs thereof wherein said homologs have at least 70, 80, 85, 90, 95, 97, 98 or 99% identity to the nucleic acids 1554 to 1958 of SEQ ID NO:**1**;
(b) a nucleic acid molecule encoding the HDAC binding fragment of APPL, said HDAC binding fragment having the amino acid sequence SEQ ID NO:**3**;
(c) a nucleic acid molecule encoding the APPL binding region of HDAC 1 consisting of the nucleic acids 235 to 336 of SEQ ID NO:**4** as well as homologs thereof wherein said homologs have at least 70, 80, 85, 90, 95, 97, 98 or 99% identity to said fragment of SEQ ID NO:**4**;
(d) a nucleic acid molecule encoding the APPL binding region of HDAC, having the amino acid sequence SEQ ID NO:**7** or homologs thereof wherein said homologs have at least 70, 80, 85, 90, 95, 97, 98 or 99% identity to SEQ ID No:**7**;
(e) a nucleic acid molecule which is complementary to the polynucleotide of (a) to (d);
(f) a nucleic acid molecule comprising at least 15 sequential bases of the polynucleotide of (a) to (e); or
(g) a nucleic acid molecule that hybridizes under stringent conditions to the polynucleotide molecule of (a) to (f),
may be useful
A vector comprising the isolated nucleic acid molecules as defined above, as well as a host cell stably transformed with such a vector are useful for the invention. The term "vector" refers to any carrier of exogenous DNA that is useful for transferring the DNA into a host cell for replication and/or appropriate expression of the exogenous DNA by the host cell. Accordingly, in a specific embodiment said vector is an expression vector such as pGL31uc, pBLCAT5 (LMBP 2451), pGMCSFlacZ (LMBP 2979), pEGFP or pSEAPbasic (DMB 3115),wherein LMBP and DMB numbers refer to the accession numbers of these expression vectors at the Belgian Co-ordinated Collections of Microorganisms. Included in the invention is also a host cell harboring a vector according to the invention. Such a host cell can be a prokaryotic cell, a unicellular eukaryotic cell or a cell derived from a multicellular organism. The host cell can thus e.g. be a bacterial cell, such as an *E. coli* cell; a yeast cell, such as *Saccharomyces cerevisiae* or *Pichia pastoris,* or a mammalian cell, such as HEK293 cells. The methods employed to effect introduction of the vector into the host cell are standard methods, well known to a person familiar with recombinant DNA methods.

For the HDAC and/or APPL protein used in the invention a vector comprising an isolated and purified nucleic acid molecule selected from a group consisting of:
(a) a nucleic acid molecule encoding the HDAC binding fragment of APPL consisting of the nucleic acids 1554 to 1958 of SEQ ID NO:**1** as well as homologs thereof wherein said homologs have at least 70, 80, 85, 90, 95, 97, 98 or 99% identity to the nucleic acids 1554 to 1958 of SEQ ID NO:**1**;
(b) a nucleic acid molecule encoding the HDAC binding fragment of APPL, said HDAC binding fragment having the amino acid sequence SEQ ID NO:3 or homologs thereof wherein said homologs have at least 70, 80, 90, 95, 96, 97, 98, or 99% sequence identity to SEQ ID NO:3
(c) a nucleic acid molecule encoding the histone deacetylase domain of HDAC1 consisting of the nucleic acids acids 109 to 1047 of SEQ ID NO:4 as well as homologs thereof wherein said homologs have at least 70, 80, 85, 90, 95, 96, 97, 98 or 99% identity to SEQ NO**:4;**
(d) a nucleic caid molecule encoding the histone deacetylase domain of HDAC 1 having the amino acid sequence SEQ ID NO:**6** or homologs thereof wherein said homologs have at least 70, 80, 90, 95, 96, 97, 98, or 99% sequence identity to SEQ ID NO:**6**;
(e) a nucleic acid molecule encoding the APPL binding region of HDAC 1 consisting of the nucleic acids 235 to 336 of SEQ ID NO:**4** as well as homologs thereof wherein said homologs have at least 70, 80, 85, 90, 95, 97, 98 or 99% identity to said fragment of SEQ ID NO:**4**;
(f) a nucleic acid molecule encoding the APPL binding region of HDAC, having the amino acid sequence SEQ ID NO:**7** or homologs thereof wherein said homologs have at least 70, 80, 85, 90, 95, 97, 98 or 99% identity to SEQ ID No:**7**;
(g) a nucleic acid molecule comprising at least 15 sequential bases of the polynucleotide of (a) to (f); or
(h) a nucleic acid molecule that hybridizes under stringent conditions to the polynucleotide molecule of (a) to (f),
may be useful.

The vectors according to the invention are particularly useful in a method to identify compounds that modulate the interaction of APPL with HDAC proteins; such methods include for example, the two-hybrid vector system, which is well known to molecular biologists (Fields & Song, Nature 340:245 1989). This technique is based on functional reconstitution in vivo of a transcription factor, which activates a reporter gene. More particularly the technique comprises providing an appropriate host cell with a DNA construct comprising a reporter gene under the control of a promoter regulated by a transcription factor having a DNA binding domain and an activating domain, expressing in the host cell a first hybrid DNA sequence encoding a first fusion of a fragment or all of a nucleic acid sequence encoding APPL or a HDAC binding fragment thereof and either said DNA binding domain or said activating domain of the transcription factor, expressing in the host at least one second hybrid DNA sequence, encoding HDAC or the APPL fragment thereof together with the DNA binding or activating domain of the transcription factor which is not incorporated in the first fusion; detecting any binding of the proteins to be investigated with a protein according to the invention by detecting for the presence of any reporter gene product in the host cell.

An example of such a technique utilises the GAL4 protein in yeast. GAL4 is a transcriptional activator of galactose metabolism in yeast and has a separate domain for binding to activators upstream of the galactose metabolising genes as well as a protein binding domain. Nucleotide vectors may be constructed, one of which comprises the nucleotide residues encoding the DNA binding domain of GAL4. These binding domain residues may be fused to a nucleic acid sequence encoding APPL or an HDAC binding fragment thereof as defined hereinbefore. The other vector comprises the residues encoding the protein binding domain of GAL4. fused to residues encoding HDAC or an APPL binding fragment thereof as defined hereinbefore. Any interaction between neurotrophic factor encoded by the nucleic acid according to the invention and the protein to be tested leads to transcriptional activation of a reporter molecule in a GAL-4 transcription deficient yeast cell into which the vectors have been transformed. Preferably, a reporter molecule such as β-galactosidase is activated upon restoration of transcription of the yeast galactose metabolism genes.

### Polypeptides

The present invention also relates to the use of HDAC enzymes or fragments thereof in an assay that makes use of the interaction of APPL and the related peptides with the HDAC enzymes, said polypeptide may be encoded by an isolated and purified nucleic acid molecule as defined above.
The HDAC enzyme as used therein consists of the HDAC enzyme or the APPL binding fragment thereof selected from the group consisting of;
(a) an isolated and purified HDAC1 protein having the amino acid sequence SEQ ID NO: **5**;
(b) an isolated and purified APPL binding fragment having the amino acid sequence SEQ ID NO: **6**;
(c) an isolated and purified APPL binding fragment having the amino acid sequence SEQ ID NO: **7**;

In one embodiment the HDAC1 protein comprises the amino acid sequence of SEQ ID NO:**5** or a fragment thereof. In particular a functional fragment thereof wherein a functional fragment of HDAC 1 retains the enzymatic activity of said protein, i.e. removal of acetyl modifications of lysine residues in proteins, including the core nucleosomal histones H2A, H2B, H3 and H4. Functional fragments of HDAC proteins would minimally comprise the histone deacetylase domain of said proteins, in particular the histone deacetylase domain of HDAC 1 having the amino acid sequence SEQ ID NO:**6** or homologs thereof wherein said homologs have at least 70, 80, 85, 90, 95, 96, 97, 98, or 99% sequence identity to SEQ ID No.**6**. In a more particular embodiment the functional fragment of the HDAC protein would consist of the isolated and purified polypeptide having the amino acid sequence SEQ ID NO:**6** or homologs thereof wherein said homolgs have at least 70, 80, 85, 90, 95, 96, 97, 98 or 99% sequence identity to SEQ ID NO:**6**. In another embodiment the HDAC1 protein consists of the amino acid sequence of SEQ ID NO:**5** or the APPL binding fragment thereof. The APPL binding fragment either consists of SEQ ID NO:7 or comprises the consensus sequence as defined hereinbefore. In particular, the APPL binding fragment either consists of SEQ ID NO:**7** or homologs thereof as defined hereinbefore.

The present invention relates to the use of APPL and the related peptides in an assay according to the invention, wherein the APPL or APPL-related proteins are selected from the group consisting of;
i) an isolated polypeptide encoding APPL, comprising the amino acid sequence SEQ ID NO:**2**;
ii) an isolated polypeptide encoding the HDAC binding fragment of APPL comprising the amino acid sequence SEQ ID NO:**3**.

In one embodiment of the present invention, APPL consists of the amino acid sequence encoded by SEQ ID NO: **2**, an APPL-related protein as defined hereinbefore, or of the HDAC binding fragment encoded by SEQ ID NO:**3**.
Given the identification and the characterization of the HDAC binding fragment within APPL and the APPL binding fragment within HDAC, it is also an object of the present invention to provide an isolated and purified APPL binding fragment having the amino acid sequence SEQ ID NO: **7**

The receptor protein and the peptides according to the invention includes all possible conservative amino acid changes, wherein "conservative amino acid changes" refers to a replacement of one or more amino acid residue(s) in a parent receptor protein or peptide without affecting the biological activity of the parent molecule based on the art recognized substitutability of certain amino acids (See e.g. M. Dayhoff, In Atlas of Protein Sequence and Structure, Vol. 5, Supp. 3, pgs 345-352, 1978).

Those skilled in the art will recognize that the polypeptides according to the invention, i.e. the HDAC enzymes, the APPL binding fragment and APPL or APPL-related proteins, could be obtained by a plurality of recombinant DNA techniques including, for example, hybridization, polymerase chain reaction (PCR) amplification, or *de novo* DNA synthesis (See e.g., T. Maniatis et al. Molecular Cloning: A Laboratory Manual, 2d Ed. Chap. 14 (1989)).

The peptides and derivatives of the present invention can be readily prepared according to well-established, standard liquid or, preferably, solid-phase peptide synthesis methods, general descriptions of which are broadly available, or they may be prepared in solution, by the liquid phase method or by any combination of solid-phase, liquid phase and solution chemistry.
A polypeptide according to the present invention may be isolated and/or purified (e.g. using an antibody) for instance after production by expression from encoding nucleic acid. The isolated and/or purified polypeptide may be used in formulation of a composition, which may include at least one additional component, for example a pharmaceutical composition including a pharmaceutically acceptable excipient, vehicle or carrier.
A polypeptide according to the present invention may be used as an immunogen or otherwise in obtaining specific antibodies. Antibodies are useful in purification and other manipulation of polypeptides, diagnostic screening and therapeutic contexts. Antibodies to the polypeptides of the present invention may, advantageously, be prepared by techniques which are known in the art. For example, polyclonal antibodies may be prepared by inoculating a host animal such as a mouse with the growth factor or an epitope thereof and recovering immune serum. Monoclonal antibodies may be prepared according to known techniques such as described by Kohler R. and Milstein C., Nature (1975) 256, 495-497.
A polypeptide according to the present invention may be used in screening for molecules which bind to it or modulate its activity or function. Such molecules may be useful in a therapeutic (possibly including prophylactic) context.
A polypeptide or labelled polypeptide of the invention or fragment thereof may also be fixed to a solid phase, for example the surface of an immunoassay well or dipstick.
Such labelled and/or immobilized polypeptides may be packaged into kits in a suitable container along with suitable reagents, controls, instructions and the like.
Such polypeptides and kits may be used in methods of detection of antibodies to such polypeptides present in a sample or active portions or fragments thereof by immunoassay.
Immunoassay methods are well known in the art and will generally comprise:
(a) providing a polypeptide comprising an epitope bindable by an antibody against said protein;
(b) incubating a biological sample with said polypeptide under conditions which allow for the formation of an antibody-antigen complex; and
(c) determining whether antibody-antigen complex comprising said polypeptide is formed.

### Assays

Assays of the present invention can be designed in many formats generally known in the art of screening compounds for biological activity or for binding proteins.

Polypeptides of the present invention are responsible for one or more biological functions, including one or more disease states, in particular the diseases hereinbefore mentioned. It is therefore desirable to devise screening methods to identify compounds which stimulate or which inhibit the function of HDACs.

The assays of the present invention advantageously exploit the fact that APPL or APPL-related proteins are co-factors for HDAC enzymes and activate HDAC enzymes upon binding thereto.

Therefore, the present invention includes methods of identifying compounds that specifically bind to HDAC enzymes, wherein said compounds may be agonists or antagonists of the HDAC enzymes. The assay methods of the present invention differ from those described in the art because the present assays incorporate at least one step wherein the interaction of APPL or APPL-related proteins with the HDACs is incorporated in the assay, or in that they apply the APPL binding fragments of HDACs.

Thus, the present invention provides for a method of identifying and obtaining a test compound capable of binding a HDAC enzyme comprising:
a) incubating a source containing a HDAC enzyme or a fragments thereof, with
   i) APPL or APPL-related proteins
   ii) said test compound; and
b) measuring the effect of the test compound on the amount of APPL or APPL-related proteins bound to the enzyme.

In a preferred embodiment, the present invention provides for a method of identifying and obtaining a test compound capable of binding HDAC1 enzyme comprising:
a) incubating a source containing HDAC1 or a fragments thereof, with
   i) APPL or APPL-related proteins
   ii) said test compound; and
b) measuring the effect of the test compound on the amount of APPL or APPL-related proteins bound to the enzyme.

The HDAC1 containing source is selected from the group consisting of;
(a) an isolated and purified HDAC 1 protein having the amino acid sequence SEQ ID NO: **5**;
(b) an isolated and purified APPL binding fragment having the amino acid sequence SEQ ID NO: **6**;
(c) an isolated and purified APPL binding fragment having the amino acid sequence SEQ ID NO: **7**.

Based upon the identification of the APPL-binding region, the present invention further provides assays to identify compounds that modulate the interaction of APPL or APPL-related proteins with said APPL binding region. Such compounds may be useful as agonists or antagonists to modulate the interaction of APPL with other enzymes, such as for example AKT2, DCC, FSHR and Rab5, comprising said APPL-binding region.

Thus, the present invention provides for a method of identifying and obtaining a test compound capable of modulating the interaction of APPL or APPL-related proteins with the APPL-binding region comprising:
a) incubating a source containing an APPL-binding region, with
   i) APPL or APPL-related proteins
   ii) said test compound; and
b) measuring the effect of the test compound on the amount of APPL or APPL-related proteins bound to the binding region.

In a further embodiment of the present invention, the source containing the APPL-binding region is selected from the group consisting of;
(a) an isolated and purified APPL binding fragment having the amino acid sequence SEQ ID NO: **6**;
(b) an isolated and purified APPL binding fragment having the amino acid sequence SEQ ID NO: **7**.

The screening method may simply measure the binding of a candidate compound to the polypeptide, or to cells or membranes bearing the polypeptide, or a fusion protein thereof by means of a label directly or indirectly associated with the candidate compound. Alternatively, the screening method may involve competition with a labeled competitor. In a preferred embodiment, this labeled competitor is a ligand known to bind to HDAC such as APPL or APPL-related proteins. In a further embodiment said APPL-related protein consists of the HDAC binding fragment encoded by SEQ ID NO:**3**.

Therefore, in a more preferred embodiment, the screening method comprises labeled APPL or labeled APPL-related proteins, wherein said label is used to measure the effect of the test compound on the amount of APPL or APPL-related protein bound to the HDAC enzyme or an APPL binding fragment thereof.

Accordingly, the present invention provides a method of identifying and obtaining a test compound capable of binding the HDAC1 enzyme comprising:
i) incubating the HDAC 1 enzyme or an APPL binding fragment thereof with a labeled APPL-related protein comprising an amino acid sequence encoded by SEQ ID NO:**2**, preferably iodinated APPL-related protein consisting of SEQ ID NO:**3**;
ii) adding the test compound to the incubation mixture; and
iii) measuring the effect of the test compound on the amount of labeled APPL-related peptide bound to the HDAC 1 enzyme or the APPL binding fragment thereof.
In one embodiment the HDAC1 enzyme has the amino acid sequence SEQ ID NO: **5**, alternatively the APPL binding fragment comprises SEQ ID NO:**7**. In another embodiment the APPL binding fragment consists of SEQ ID NO:7 or comprises the consensus sequence as defined hereinbefore.

Examples of possible binding assays are the immunoprecipitation assay as provided in the examples hereinafter or the use of a surface plasmon resonance effect exploited by the Biacore instrument (Malmqvist M., Biochem Soc Trans. 1999 Feb;27(2):335-40). In the latter FLAG-tagged or His-tagged version of the polypeptides of this invention could be attached to the biosensor chip of a Biacore and binding of binding partner examined in the presence and absence of compounds to identify competitors of the binding site. For example, in one embodiment the HDAC-binding fragment of APPL or homologs thereof as defined hereinbefore would be immobilized on the Biacore chip using a Flag tag and the binding of HDAC or the APPL binding fragment thereof would be examined in the presence and absence of compounds to identify competitors of the binding site. Alternatively, the APPL-binding fragment of HDAC or homologs thereof as defined hereinbefore would be immobilized on the Biacore chip using a Flag tag and the binding of APPL or the HDAC binding fragment thereof would be examined in the presence and absence of compounds to identify competitors of the binding site.
Tagging of the polypeptides according to the invention, is also useful to immobilize said molecules in conventional filter-binding assays (eg. Using Brandel filter assay equipment) or in high throughput Scintillation Proximity type binding assays (SPA and Cytostar-T flashplate technology; Amersham Pharmacia Biotech) to detect binding of radio-labelled ligand and displacement of such radio-ligands by competitors for the binding site. Radioactivity can be measured with Packard Topcount, or similar instrumentation, capable of making rapid measurements from 96-, 384-, 1536-microtitre well formats. SPA/Cytostar-T technology is particularly amenable to high throughput screening and therefore this technology is suitable to use as a screen for compounds able to displace standard ligands.

Further, these screening methods may test whether the candidate compound results in a signal generated by activation or inhibition of the enzyme, using detection systems appropriate to enzymatic activity of said enzyme. Enzymatic activity is generally assessed using an appropriate substrate that upon processing provides a measurable signal. Assays to measure the activity/activation of HDACs are generally known in the art and include amongst others radioactive-labeled or fluorescent-labeled acetylated histone as a substrate and measuring the release of the labelled acetyl group such as for example provide in Example 4 hereinafter.

Therefore, the present invention provides a method of identifying and obtaining a test compound capable of modulating the activity of HDACs comprising:
a) incubating HDAC or functional fragments thereof, with said test compound;
b) measuring the effect of the test compound on the activity of the HDAC enzyme; and
c) compare this effect with the activity of the HDAC enzyme upon binding of APPL or APPL-related proteins.

The HDAC enzyme is selected from the group consisting of;
a) HDAC1 having amino acid sequence SEQ ID NO:**5**;
b) the histone deacetylase domain of HDAC 1 having the amino acid sequence SEQ ID NO:6.

The effect of the test compound on the HDAC enzyme is typically assessed using radioactive-labeled or fluorescent-labeled acetylated histone as a substrate and measuring the release of the labelled acetyl group. In particular using the radioactive-labeled acetylated histone H4 peptide as provided in Example 4 hereinafter.

It will be readily appreciated by the skilled artisan that the discovery of the interaction of APPL or APPL-related proteins with HDAC may also be used in a method for the structure-based or rational design of an agonist or antagonist of HDAC, by:
a) probing the structure of the binding site on HDAC with APPL or APPL derivatives;
b) identifying contacting atoms in the binding site of the HDAC protein that interact with the APPL ligand during binding;
c) design test compounds that interact with the atoms identified in (b) to modulate the activity of the HDAC enzyme; and
d) contact said designed test compound with a HDAC or a functional fragment thereof, to measure the capability of said compound to modulate the HDAC activity.
It will be further appreciated that this will normally be an iterative process.
This invention further provides a method for evaluating the potential of a test compound to interact with the APPL binding site said method comprising;
(a) using molecular modeling techniques to formulate a three dimensional structure of the APPL binding site;
(b) employing computational means to perform a fitting operation between the test compound and the three-dimensional structure of the APPL binding site; and
(c) analyzing the results of said fitting operation to quantify the association of the test compound with the three dimensional structure of the APPL binding site.
Molecular modeling techniques are known in the art, including both hardware and software appropriate for creating and utilizing models of receptors and enzyme conformations.
Numerous computer programs are available and suitable for the processes of computer modeling, model building and computationally identifying, selecting and evaluating potential atpE interacting compounds in the methods described herein. These include for example, GRID (available from Oxford University, UK), MCSS (available from Accelrys, Inc., San Diego, CA), AUTODOCK (available from Oxford Molecular Group), FLEX X (available form Tripos, St. Louis. MO), DOCK (available from University of California, San Francisco, CA), CAVEAT (available from University of California, Berkeley), HOOK (available from Accelrys, Inc., San Diego, CA) and 3D database systems such as MACCS-3D (available from MDL Information Systems, San Leandro, CA), UNITY (available from Tripos, St. Louis.MO) and CATALYST (available from Accelrys, Inc., San Diego, CA). Potential candidate substances may also be computationally designed *"de novo'* using software packages as LUDI (available from Biosym Technologies, San Diego, CA), LEGEND (available from Accelrys, Inc, San Diego, CA) and LEAPFROG (available from Tripos, St. Louis.MO). Compound deformation energy and electrostatic repulsion, may be analysed using programs such as GAUSSIAN 92, AMBER, QUANTA/CHARMM and INSIGHT II/DISCOVER. These computer evaluation and modeling techniques may be performed on any suitable hardware including for example, workstations available from Silicon Graphics, Sun Microsystems and others. These modeling techniques, methods, hardware and software packages are representative and are not intended to be a comprehensive listing. Other modeling techniques known in the art may also be employed in accordance with this invention. See for example, N.C. Cohen, Molecular Modeling in Drug Design, Academic Press (1996).
In one embodiment of the present invention, the three-dimensional structure of the APPL binding site is generated using the atomic coordinates of the HDAC8 protein (Protein Database 1W22) +/- a root mean square deviation of the backbone atoms of said amino acids of not more that 10Å, preferably not more that 5 Å.

### Therapeutic use

In general, agonists or antagonists may be employed for therapeutic and prophylactic purposes for such diseases as hereinbefore mentioned. Compounds may be identified from a variety of sources, for example, cells, cell-free preparations, chemical libraries, and natural product mixtures. Such agonists or antagonists so-identified may be natural or modified peptides, ligands, enzymes, etc., as the case may be, of the receptor polypeptide; or may be structural or functional mimetics thereof (see Coligan et al., Current Protocols in Immunology 1 (2):Chapter 5 (1991)).

Therefore, the present invention relates to the use of the APPL binding fragment or homologs thereof as a medicine and for use in the treatment of inhibiting the growth of tumours. Examples of tumours which may be inhibited, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumours of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumours of mesenchymal origin (e.g. fibrosarcomas and rhabdomyosarcomas), melanomas, teratocarcinomas, neuroblastomas, gliomas, benign tumour of the skin (e.g. keratoacanthomas), breast carcinoma (e.g. advanced breast cancer), kidney carcinoma, ovary carcinoma, bladder carcinoma and epidermal carcinoma.

The APPL binding fragment consists of SEQ ID NO:**7**.

Therefore, the present invention may identify a compound in an assay according to the invention, wherein said compound is capable of binding and/or modulating the HDAC enzymatic activity and wherein said compound is either an agonist or antagonist of the enzyme as determined in any of the above described assays. Said compounds may be used as a medicine and may be used in the treatment of inhibiting the growth of tumours. Examples of tumours which may be inhibited, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumours of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumours of mesenchymal origin (e.g. fibrosarcomas and rhabdomyosarcomas), melanomas, teratocarcinomas, neuroblastomas, gliomas, benign tumour of the skin (e.g. keratoacanthomas), breast carcinoma (e.g. advanced breast cancer), kidney carcinoma, ovary carcinoma, bladder carcinoma and epidermal carcinoma.

Thus, in a further aspect, the present invention provides a method for preventing, treating or ameliorating a medical condition related to a disorder of HDAC activity which comprises administering to a mammalian subject a therapeutically effective amount of APPL-related proteins and the APPL binding fragment, optionally in combination with a pharmaceutically acceptable carrier, in an amount effective to modulate the HDAC enzymatic activity. Such carriers include, but are not limited to, saline, buffered saline, dextrose, water, glycerol, ethanol, and combinations thereof. Pharmaceutical packs and kits comprising one or more containers filled with one or more of the ingredients of the aforementioned compositions of the invention are envisioned. Polypeptides and other compounds of the present invention may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide of the present invention can be formulated in an enteric or an encapsulated formulation, oral administration may also be possible. Administration of these compounds may also be topical and/or localized, in the form of patches, salves, pastes, gels, and the like.

The dosage range required depends on the choice of peptide or other compounds of the present invention, the route of administration, the nature of the formulation, the nature of the subject's condition, and the judgment of the attending practitioner. Suitable dosages, however, are in the range of 0.01 mg/kg to 300 mg/kg body weight, in particular from 5 mg/kg to 150 mg/kg body weight, even more particular from 2 mg/kg to 100 mg/kg body weight. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art. Generally, an amount of an HDAC modulator to be administered as a therapeutic agent for treating cell proliferative disorder such as atherosclerosis, restenosis and cancer, will be determined on a case by case by an attending physician.

This invention will be better understood by reference to the Experimental Details that follow, but those skilled in the art will readily appreciate that these are only illustrative of the invention as described more fully in the claims that follow thereafter. Additionally, throughout this application, various publications are cited. The disclosure of these publications is hereby incorporated by reference into this application to describe more fully the state of the art to which this invention pertains.

### Example 1: Identification of APPL as a novel HDAC1 binding protein

In order to identify novel HDAC1 associated proteins, a yeast two-hybrid screen was performed using the full length HDAC1 as bait against a human brain cDNA library This resulted in the identification of a fragment of a PTB domain-containing protein (aa 489-639), previously described as APPL⁷ or DIP13α⁸, an adaptor protein that may exert an important function in cell signaling. The identified fragment (aa 489-639) of APPL fully overlapped the PTB domain of APPL (aa 500-634) and will be further referred to as APPL-PTB.

### Example 2: lnteraction of HDAC1 and APPL

To determine whether the interaction between HDAC1 and the fragment of APPL (amino acids 489-639) also occurred in eukaryotic cells, a series of coimmunoprecipitation assays was performed in HEK293 cells. As shown in figure 1A, APPL-PTB was coimmunoprecipitated with HDAC1 when both proteins were overexpressed (lane 4). This interaction is specific since no complex was detected in the absence of HDAC1 overexpression (figure 1A, lanes 2-4). The reverse immunoprecipitation, *i.e.* of APPL-PTB also resulted in the coimmunoprecipitation of HDAC1 (data not shown). The fact that the PTB domain of APPL alone already showed an efficient coimmunoprecipitation with HDAC1, indicates that this domain is sufficient for the interaction. PTB domains were originally found to interact with phosphorylated NPXpY motifs, a motif that is not present in HDAC1. However, recent publications indicate that PTB binding is not stringency dependent on the NPXpY motif^{12,13}.
Subsequently, we confirmed the interaction between HDAC1 and the full length APPL protein. V5-APPL was coimmunoprecipitated with HDAC1-flag when both of these proteins were cotransfected (lane 4, figure 1B). In conclusion, we can state that overexpressed HDAC1 interacts with both the isolated PTB domain of APPL as well as with the full-length protein. Interestingly, up to date, all proteins described to bind to APPL, *i.e.* Akt2, DCC, FSHR and Rab5 ⁷⁻¹⁰ interact through the APPL PTB (phosphotyrosine binding) domain. We therefore aligned all APPL-PTB binding partners and identified a strong consensus sequence, which is located between amino acid residues 51 and 88 of HDAC1 (Figure 5) and comprises SEQ ID No.7. Interestingly, the same consensus was identified in all cass I HDACs, including HDAC2, HDAC3 and HDAC8, but not in any of the class II or class III family members. The fact that numerous signal transduction proteins bind to APPL points at a possible role for APPL as a scaffolding protein. Since the PTB domain of APPL is involved in each interaction, this suggests that different binding partners may compete with each other. We found that overexpression of Akt2 blocked the coimmunoprecipitation of APPL with HDAC1 (data not shown), indicating that HDAC 1 and Akt2 compete to bind APPL.

### Example 3: Endogenous HDAC1 and APPL co-localize.

To determine whether the endogenous APPL and HDAC1 to interact, we have investigated the localization of APPL and HDAC1 in human MDA-MB-231 breast carcinoma and A2780 ovarian carcinoma cells using immunocytochemistry. Figure 2 shows the colocalization of HDAC1 and APPL in cells during interphase. APPL staining shows a granular structure, which is predominantly present in the cytoplasm, which has previously also been described by Testa et al ⁷. A small proportion of the APPL vesicles, however, is localized in the nucleus where they overlap with HDAC 1. Confocal 3D analysis of the co-localization of endogenous APPL and HDAC1 signals showed a clear overlap of APPL and HDAC1 inside the nucleus (figure 2, pixel intensity graphs). This shows that the proteins have indeed a possibility to interact in quiescent cells. In order to evaluate how these two proteins may interact during cell cycle progression, we evaluated the localization of APPL and HDAC1 in cells in mitosis (Figure 6). During metaphase, HDAC1 reorganizes around the chromosomes, while APPL is present more diffusely. In anaphase, HDAC1 is still present around the chromosomes. Interestingly, during this particular stage of mitosis, APPL seems to be localized predominantly at the same spot as HDAC1: in-between the sister chromatides that are being separated, as evident by a dominant yellow region. At later stages, both APPL and HDAC 1 are more diffusely expressed. During late telophase and cytokinesis, APPL and HDAC 1 redistribute. HDAC1 is in this stage only present in the nucleus, while APPL is present predominantly in the cytoplasm, so no overlap could be detected during late telophase and cytokinesis. The colocalization experiments in MDA-MB-231 cells showed comparable results; APPL is more granularly structured, and there seems to be more APPL in the nucleus of MDA-MB-231 cells during interphase than in A2780 cells. In A2780 cells, more HDAC 1 appears to be present in the cytoplasm. The fact that under unstimulated conditions in cells in interphase only a minor fraction of APPL colocalizes with HDAC 1 may explain why the coimmunoprecipitation and HDAC 1 activity experiments were technically challenging and depended on the extend of APPL overexpression. Interestingly, Miaczynska et al. (2004)⁹ showed that APPL translocates to the nucleus upon EGF stimulation in HeLa cells, suggesting that after activation of the EGF signalling pathway, larger amounts of APPL and HDAC 1 may interact. In conclusion we can state that endogenous APPL and HDAC1 clearly co-localize, emphasizing a physiological relevance for the observed interaction.

### Example 4: APPL increases HDAC1 activity in human HEK293 cells.

Since HDAC1 and APPL directly interact in HEK293 cells, we wondered whether APPL had an effect on HDAC1 activity. Both proteins were equally overexpressed in HEK293 cells (figure 3B). After 48 hours of overexpression, cells were lysed, HDAC1 was immunoprecipitated and activity was assessed by determining the release of acetic acid from an [³H]acetyl-labeled Histone H4 peptide. Remarkably, in the presence of overexpressed APPL, HDAC1 activity was approximately 4-fold increased (figure 3A, compare conditions 2 and 4), even though equal amount of HDAC1 were immunoprecipitated (figure 3B, top panel). These results indicate that APPL is a novel regulator of HDAC1 enzymatic activity. How HDAC1 activity is increased by APPL, however, is currently still unclear. Based on the known regulatory mechanisms of HDAC activity, this may involve either stabilization of the core HDAC-NuRD complex and/or potentiating HDAC1 posttranslational modifications.

### Example 5: Overexpressed APPL decreases acetylation levels of histone H3 in HEK293 cells

In order to further validate our hypothesis that APPL increases HDAC 1 activity, the acetylation status of histone H3 was determined. We hypothesized that if APPL had an effect on HDAC1 activity, the acetylation levels of histone H3 should decrease after APPL overexpression. Therefore, HEK293 cells were transfected with APPL and after 48 hours, the cells were enriched for the transfected population using the pMACS selection system (see experimental procedures). Total protein extracts were prepared and analyzed on Western blot. After 48 hours of APPL overexpression, a clear decrease in acetylation status of histone H3 could be detected, compared to the mock control (figure 4, compare lanes 2 and 4), while the total H3 protein level remained equal in all lanes. Also, as expected, overexpression of HDAC1 results in a decrease of H3 acetylation, indicating that the amount of endogenous HDAC1 activity present is rate-limiting. The presence of both overexpressed proteins did not further decrease acetylation levels of H3 (Figure 4A, lanes 5), but it should be noted that H3 acetylation was already surpressed with HDAC1 or APPL alone in this experiment (Figure 5A, lanes 3 and 4). This result shows that APPL decreases the acetylation status of histone H3, which is in line with an increased HDAC1 activity.

### Example 6: Overexpressed APPL decreases p21^{waf1,cip1} protein levels in HEK293 cells

HDAC1 activity is crucial in maintaining low expression levels of the cyclin dependent kinase inhibitor p21^{*waf1,cip1* 2,11}. The p21*^{waf1,cip1}* promotor contains several Sp1 binding sites, through which HDAC1 can be recruited. We hypothesized that if APPL specifically increases HDAC1 activity and decreases the H3 acetylation status, p21*^{waf1,cip1}* protein levels should drop upon APPL overexpression. Again, HEK293 cells were transfected with HDAC1 and /or APPL during 48 hours. Total protein extracts were prepared and analyzed on Western blot. When APPL was overexpressed, almost a complete suppression in p21*^{waf1,cip1}* protein levels could be detected, compared to the control (figure 4B, compare lanes 1 and 3). This effect is specific for p21*^{waf1,cip1}*, since no effect could be detected on p 16, another cyclin-dependent kinase inhibitor. To confirm that the observed APPL-mediated downregulation of p21*^{waf1,cip1}* is dependent on the endogenous HDAC1 activity, we have utilized a HDAC1 mutant (HDAC1-H141A). In this mutant histidine 141 is replaced by an alanine residue and shows 50% reduction in enzymatic activity (Hassig et al., PNAS 95; 3519-3524 1998). HDAC1-H141A, however, still has the ability to form the Sin3A chromatin remodelling comples. As illustrated in Figure 4B, the coexpression of the H141A mutant partially reverses the suppression of p21*^{waf1,cip1}* by APPL (compare lanes 4 and 6), demonstrating a key role for HDAC1 in the APPL mediated suppression of p21*^{waf1,cip1}.*

### Conclusion

In this screen, we have identified APPL (adaptor protein containing PH domain, PTB domain, and Leucine zipper motif) as a novel interaction factor of HDAC 1. APPL, also known as DIP13α, stands for "adaptor protein containing a PH domain, a PTB domain and Leucine zipper motif"⁷⁻¹⁰. Although APPL was originally described as a cytoplasmic protein⁷, it was recently described to translocate to the nucleus upon stimulation with EGF⁹ where it temporarily interacts with the NuRD/Mi2 complex. There are several indications that APPL plays an important role in cell cycle regulation and apoptosis. First, APPL has been shown to interact with the oncogene Akt2⁷. Second, it was described to interact with the pro-apoptotic protein DCC (deleted in colorectal cancer). Overexpression of APPL enhances DCC-induced cell death⁸. Third, Miaczynska et al. showed recently that knocking down APPL using siRNA has inhibitory effects on DNA synthesis⁹. Although APPL clearly affects cell cycle progression, the mechanism by which this occurs is currently not known.

In this paper we show that APPL is a novel direct interactor of HDAC 1 and a key factor in the regulation of its enzymatic activity. Overexpression of APPL results in an increase of HDAC 1 activity and a subsequent decreased acetylation status of histone H3. Furthermore, APPL overexpression causes a decreased basal protein level of p21*^{waf1,cip1}*, an important G1 cell cycle checkpoint regulator which expression levels are strictly dependent on HDAC1 activity.

Summarized, we have found that APPL potentiates HDAC 1 activity, and modulates HDAC1 downstream functions. Since HDAC1 activity is crucial for tumor cell proliferation, this illustrates an oncogenic and/or pro-proliferative role for APPL. Interestingly, Miaczynska et al. (2004)⁹ showed with siRNA experiments that after knock down of APPL in HeLa cells, there is a clear decrease of the percentage of cells in S-phase after 48 hours. In conclusion, our data show that APPL directly interacts with HDAC1 and that APPL overexpression decreases histone H3 acetylation and p21*^{waf1,cip1}* protein levels and increases the enzymatic activity of HDAC 1. Our findings indicate that APPL is important in regulating the activity of the oncogene HDAC 1, pointing at a key role for APPL in tumor cell proliferation.

### References

1. Arts,J., de Schepper,S. & Van Emelen,K. Histone deacetylase inhibitors: from chromatin remodeling to experimental cancer therapeutics. Curr. Med Chem. 10, 2343-2350 (2003).
2. Lagger,G. et al. Essential function of histone deacetylase 1 in proliferation control and CDK inhibitor repression. EMBO J 21, 2672-2681 (2002).
3. Sengupta,N. & Seto,E. Regulation of histone deacetylase activities. J. Cell Biochem. 93, 57-67 (2004).
4. Pflum,M.K., Tong,J.K., Lane,W.S. & Schreiber,S.L. Histone deacetylase 1 phosphorylation promotes enzymatic activity and complex formation. J. Biol. Chem. 276,47733-47741 (2001).
5. Zhou,Q. et al. Rapid induction of histone hyperacetylation and cellular differentiation in human breast tumor cell lines following degradation of histone deacetylase-1. J. Biol. Chem. 275, 35256-35263 (2000).
6. David,G., Neptune,M.A. & DePinho,R.A. SUMO-1 modification of histone deacetylase 1 (HDAC1) modulates its biological activities. J Biol. Chem. 277, 23658-23663 (2002).
7. Mitsuuchi,Y. et al. Identification of a chromosome 3p 14.3-21.1 gene, APPL, encoding an adaptor molecule that interacts with the oncoprotein-serine/threonine kinase AKT2. Oncogene 18, 4891-4898 (1999).
8. Liu,J. et al. Mediation of the DCC apoptotic signal by DIP 13 alpha. J Biol. Chem. 277, 26281-26285 (2002).
9. Miaczynska,M. et al. APPL proteins link Rab5 to nuclear signal transduction via an endosomal compartment. Cell 116, 445-456 (2004).
10. Nechamen,C.A. et al. Human follicle-stimulating hormone (FSH) receptor interacts with the adaptor protein APPL1 in HEK 293 cells: potential involvement of the PI3K pathway in FSH signaling. Biol. Reprod. 71, 629-636 (2004).
11. Lagger,G. et al. The tumor suppressor p53 and histone deacetylase 1 are antagonistic regulators of the cyclin-dependent kinase inhibitor p21/WAF1/CIP1 gene. Mol. Cell Biol. 23, 2669-2679 (2003).
12. Yan,K.S., Kuti,M. & Zhou,M.M. PTB or not PTB -- that is the question. FEBS Lett. 513, 67-70 (2002).
13. Guy,G.R., Yusoff,P., Bangarusamy,D., Fong,C.W. & Wong,E.S. Dockers at the crossroads. Cell Signal. 14, 11-20 (2002).
14. Fu,C.A. et al. TNIK, a novel member of the germinal center kinase family that activates the c-Jun N-terminal kinase pathway and regulates the cytoskeleton. J Biol. Chem. 274, 30729-30737 (1999).
15. Van,d.W., I et al. Cloning and characterization of human histone deacetylase 8. FEBS Lett. 478, 77-83 (2000).

### SEQUENCE LISTING

<110> Janssen Pharmaceutica N.V.
<120> HDAC regulation assays, compounds and therapeutic compositions
<130> PRD2483
<150> EP05104907.0
   <151> 2005-06-06
<150> EP05110148.3
   <151> 2005-10-28
<160> 7
<170> PatentIn version 3.1
<210> 1
   <211> 3042
   <212> DNA
   <213> homo sapiens
<220>
   <221> CDS
   <222> (57)..(2186)
   <223>
<220>
   <221> misc_feature
   <222> (1554) .. (1958)
   <223> PTB Domain / HDAC binding fragment
<400> 1
   cgcggggagc tgtgggcggc agctgcgtct cctgccactg ccctccctcc gccacgatgc      60
   cggggatcga caagctgccc atcgaggaga cgctggagga cagcccgcag acaaggtctt     120
   tactaggtgt atttgaagaa gatgccacag ctatttccaa ctatatgaac cagttgtatc     180
   aagctatgca tcggatttat gatgcacaga atgaattaag tgcagcaaca cacctgacct     240
   caaaactttt aaaagaatat gaaaaacagc gttttccatt gggaggtgat gatgaagtta 300
   tgagctctac attgcaacag ttttcaaaag ttatagatga gcttagctct tgtcatgcag 360
   tgctttcaac tcaacttgct gatgccatga tgttccccat tacccagttt aaagaaagag 420
   atctgaaaga aatactaaca ttaaaggaag tatttcagat tgcaagtaat gatcatgatg 480
   ctgcgattaa tagatatagc cgtttatcaa aaaaaagaga aaatgacaag gtgaagtatg 540
   aagtaacaga agatgtgtac acatccagaa agaaacaaca ccagaccatg atgcattatt 600
   tttgtgcatt aaatactctt cagtacaaga agaaaatagc attgttagaa cctctacttg 660
   ggtacatgca agctcagata agtttcttta agatgggttc tgaaaatctt aatgaacaac 720
   tggaagaatt tttagctaat attggaacaa gcgttcagaa tgttcgcagg gaaatggaca 780
   gtgatataga gaccatgcaa cagacaatag aggatttgga agtagccagt gatcccttat 840
   atgtgcctga cccagacccc accaaatttc ctgttaatcg aaatttaacc cgaaaggctg 900
   gataccttaa tgctaggaat aaaacaggct tggtgtcatc tacctgggac agacagtttt 960
   acttcacgca gggtggaaat ttaatgagtc aggcccgtgg ggatgtagca ggaggcctgg 1020
   ccatggacat agacaactgt tcagtgatgg ctgtggactg tgaagacaga cgatattgtt 1080
   ttcagatcac ctctttcgat ggaaaaaaat cttcaatttt gcaagcagag agtaaaaaag 1140
   atcatgaaga gtggatctgt acaataaaca acatatctaa acaaatatac ttaagtgaaa 1200
   atccagagga aactgctgca cgagtaaatc aatcagctct ggaagctgtc actccttccc 1260
   catctttcca gcagaggcac gagagcctgc ggccagcagc aggacaatct cggccaccga 1320
   cagctcgaac cagcagttca ggatccttag gatctgagtc tacaaatttg gctgccctct 1380
   ctctagattc tcttgttgcc ccagacaccc caatacagtt tgacataatt tctcctgtgt 1440
   gtgaagatca gcctggccag gcaaaagcct ttggccaggg aggcaggcgt acaaatccat 1500
   ttggagaatc tggaggaagt acaaaatctg aaactgaaga ttctattctt catcagttat 1560
   ttattgtccg attccttggt tcaatggagg tgaaatcaga tgaccatcca gatgttgttt 1620
   atgaaactat gcgccaaatc ttagctgccc gggccatcca taacatcttt cgtatgacag 1680
   aatcgcattt attagtcact tgtgactgtt taaagttaat tgatccacag acacaagtta 1740
   caaggctcac gtttccatta ccttgtgtag ttttgtatgc tacacaccag gaaaataagc 1800
   gcctttttgg atttgttctt cggacatcaa gcgggagaag tgaaagtaat ctgtcatcag 1860
   tctgctatat atttgagtca aacaatgagg gggaaaagat atgtgattct gttggactgg 1920
   caaaacagat agctttgcat gctgaactgg atcgtagggc atcagaaaaa caaaaagaaa 1980
   tagagagagt aaaagagaag caacagaaag aactcaataa acaaaaacag attgaaaagg 2040
   acttggaaga acaaagtcgg ttgatagctg cttccagtag accaaaccaa gccagtagtg 2100
   aggggcagtt tgttgtcctt agcagtagcc agtcagaaga gagtgatttg ggagaaggag 2160
   gaaagaagag agaatcagaa gcataagctt atacttttgg taaatattcc cccttggaat 2220
   ttgacagttt ctatggtgaa atggcagaag gtaacaacta tgttgaaata tcaaggagga 2280
   gattaagctt tatatttgcc taattgttgt agctacattt taaaaaaaag attgaacttg 2340
   atgacttaag tttgcattga tctttttccc ccttaaacat aatgtactat gtattaacat 2400
   cctaaaggga acctgctcat ctccctgaag cagactgctg aagaaataca tttgctcaag 2460
   aatttttccg tcaaatttgt gaacttttaa ttcctgcaat tgtaattggc tgtgcccata 2520
   ggaatcattt attcttcagg cttagacatt catggatatg cttttctttc attaggaaca 2580
   ttttgctggt gatgaggaag catttagctg aataagttta aagcccttta taccggaatt 2640
   ctacaagttt gcaaatattt taacaatatt aaatgtgcaa tagaactttt ttaaaataat 2700
   tggaacggga ttttacagtt aaggtttcaa attgtggcag gtggtactgt tgatctcagg 2760
   gtactttctg ggattgctca catttctcta atgtactgca cttgatgcca gtcggaaaga 2820
   agcttaagtg tcttcagttc aagattgata gagcccttgg cattttatta tcacattctt 2880
   agttctcagg ttgggacttc aattactgct gcagagccgt agtggttaaa aataagatat 2940
   tggaatttat taaaagattt ttgttcaata cattttagat taggattgac aagtaaagat 3000
   actgctatgg aatgtaaaaa aaaaaaaaaa aaaaaaaaaa aa 3042
<210> 2
   <211> 709
   <212> PRT
   <213> homo sapiens
<220>
   <221> misc_feature
   <222> (500)..(635)
   <223> PTB Domain / HDAC binding fragment
<400> 2
<210> 3
   <211> 135
   <212> PRT
   <213> homo sapiens
<400> 3
<210> 4
   <211> 2120
   <212> DNA
   <213> homo sapiens
<220>
   <221> CDS
   <222> (85) .. (1533)
   <223>
<220>
   <221> misc-feature
   <222> (109)..(1047)
   <223> Histone deacetylase domain
<220>
   <221> misc-feature
   <222> (235) .. (336)
   <223> APPL binding fragment
<400> 4
   ctcccccctg ggtcggacgc tgagcggagc cgcgggcggg agggcggacg gaccgactga      60
   cggtagggac gggaggcgag caagatggcg cagacgcagg gcacccggag gaaagtctgt     120
   tactactacg acggggatgt tggaaattac tattatggac aaggccaccc aatgaagcct     180
   caccgaatcc gcatgactca taatttgctg ctcaactatg gtctctaccg aaaaatggaa     240
   atctatcgcc ctcacaaagc caatgctgag gagatgacca agtaccacag cgatgactac     300
   attaaattct tgcgctccat ccgtccagat aacatgtcgg agtacagcaa gcagatgcag     360
   agattcaacg ttggtgagga ctgtccagta ttcgatggcc tgtttgagtt ctgtcagttg 420
   tctactggtg gttctgtggc aagtgctgtg aaacttaata agcagcagac ggacatcgct 480
   gtgaattggg ctgggggcct gcaccatgca aagaagtccg aggcatctgg cttctgttac 540
   gtcaatgata tcgtcttggc catcctggaa ctgctaaagt atcaccagag ggtgctgtac 600
   attgacattg atattcacca tggtgacggc gtggaagagg ccttctacac cacggaccgg 660
   gtcatgactg tgtcctttca taagtatgga gagtacttcc caggaactgg ggacctacgg 720
   gatatcgggg ctggcaaagg caagtattat gctgttaact acccgctccg agacgggatt 780
   gatgacgagt cctatgaggc cattttcaag ccggtcatgt ccaaagtaat ggagatgttc 840
   cagcctagtg cggtggtctt acagtgtggc tcagactccc tatctgggga tcggttaggt 900
   tgcttcaatc taactatcaa aggacacgcc aagtgtgtgg aatttgtcaa gagctttaac 960
   ctgcctatgc tgatgctggg aggcggtggt tacaccattc gtaacgttgc ccggtgctgg 1020
   acatatgaga cagctgtggc cctggatacg gagatcccta atgagcttcc atacaatgac 1080
   tactttgaat actttggacc agatttcaag ctccacatca gtccttccaa tatgactaac 1140
   cagaacacga atgagtacct ggagaagatc aaacagcgac tgtttgagaa ccttagaatg 1200
   ctgccgcacg cacctggggt ccaaatgcag gcgattcctg aggacgccat ccctgaggag 1260
   agtggcgatg aggacgaaga cgaccctgac aagcgcatct cgatctgctc ctctgacaaa 1320
   cgaattgcct gtgaggaaga gttctccgat tctgaagagg agggagaggg gggccgcaag 1380
   aactcttcca acttcaaaaa agccaagaga gtcaaaacag aggatgaaaa agagaaagac 1440
   ccagaggaga agaaagaagt caccgaagag gagaaaacca aggaggagaa gccagaagcc 1500
   aaaggggtca aggaggaggt caagttggcc tgaatggacc tctccagctc tggcttcctg 1560
   ctgagtccct cacgtttctt ccccaacccc tcagatttta tattttctat ttctctgtgt 1620
   atttatataa aaatttatta aatataaata tccccaggga cagaaaccaa ggccccgagc 1680
   tcagggcagc tgtgctgggt gagctcttcc aggagccacc ttgccaccca ttcttcccgt 1740
   tcttaacttt gaaccataaa gggtgccagg tctgggtgaa agggatactt ttatgcaacc 1800
   ataagacaaa ctcctgaaat gccaagtgcc tgcttagtag ctttggaaag gtgcccttat 1860
   tgaacattct agaaggggtg gctgggtctt caaggatctc ctgttttttt caggctccta 1920
   aagtaacatc agccattttt agattggttc tgttttcgta ccttcccact ggcctcaagt 1980
   gagccaagaa acactgcctg ccctctgtct gtcttctcct aattctgcag gtggaggttg 2040
   ctagtctagt ttcctttttg agatactatt ttcatttttg tgagcctctt tgtaataaaa 2100
   tggtacattt ctatatcctc 2120
<210> 5
   <211> 482
   <212> PRT
   <213> homo sapiens
<400> 5
<210> 6
   <211> 313
   <212> PRT
   <213> homo sapiens
<400> 6
<210> 7
   <211> 34
   <212> PRT
   <213> homo sapiens
<400> 7
   MEIYRPHKAN AEEMTKYHSD DYIKFLRSIR PDNM 34

## Claims

1. A method for the structure-based or rational design of an agonist or antagonist of HDAC that makes use of the interaction of an Adaptor protein containing PH domain, PTB domain, and Leucine zipper motif (APPL) or an APPL-related protein with a histone deacetylase (HDAC) enzyme, said method comprising the steps:
a) probing the structure of the ligand binding site on HDAC with APPL or APPL-related proteins;
b) identifying contacting atoms in the ligand binding site of the HDAC enzyme that interact with the APPL ligand during binding;
c) design test compounds that interact with the atoms identified in (b) to modulate the activity of the HDAC enzyme; and
d) contact said designed test compound with HDAC or a functional fragment thereof, to measure the capability of said compound to modulate the HDAC activity,
wherein the HDAC enzyme is an isolated polypeptide comprising the amino acid sequence SEQ ID NO: 5, SEQ ID NO: 6, or SEQ ID NO: 7, and wherein the APPL or APPL-related protein is an isolated polypeptide comprising amino acid sequence SEQ ID NO: 2 or SEQ ID NO: 3.

2. An isolated and purified APPL binding fragment having amino acid sequence SEQ ID NO:7.

3. An isolated and purified nucleic acid molecule encoding an APPL binding fragment encoded by nucleic acids 235 to 336 of SEQ ID NO: 4.

4. A method of identifying and obtaining a test compound capable of binding a HDAC enzyme comprising:
a) incubating a source containing a HDAC enzyme or a fragments thereof, with
i) APPL or APPL-related proteins
ii) said test compound; and
b) measuring the effect of the test compound on the amount of APPL or APPL-related proteins bound to the enzyme,
wherein the HDAC enzyme or a fragment thereof is an isolated polypeptide comprising the amino acid sequence SEQ ID NO: 5, SEQ ID NO: 6, or SEQ ID NO: 7, and wherein the APPL or APPL-related protein is an isolated polypeptide comprising amino acid sequence SEQ ID NO: 2 or SEQ ID NO: 3.

5. The method according to claim 4 wherein APPL or the APPL-related protein are labeled and wherein said label is used to measure the effect of the test compound on the amount of APPL proteins bound to the enzyme.

6. A method of identifying and obtaining a test compound capable of modulating the activity of the HDACs comprising:
a) incubating a HDAC or functional fragments thereof, with said test compound;
b) measuring the effect of the test compound on the activity of the HDAC enzyme; and
c) compare this effect with the activity of the HDAC enzyme upon binding of the APPL ligand or APPL-related proteins,
wherein the HDAC enzyme or functional fragment thereof is an isolated polypeptide comprising the amino acid sequence SEQ ID NO: 5, SEQ ID NO: 6, or SEQ ID NO: 7, and wherein the APPL ligand or APPL-related protein is an isolated polypeptide comprising amino acid sequence SEQ ID NO: 2 or SEQ ID NO: 3.

7. The method according to claim 6, wherein the effect of the test compound on the HDAC enzyme is assessed using radioactive-labeled or fluorescent-labeled acetylated histone as a substrate and measuring the release of the labeled acetyl group.

8. The method according to claim 7, wherein the substrate is radioactive-labeled acetylated histone H4 peptide.

9. The APPL binding fragment having amino acid sequence SEQ ID NO:7 for use as a medicine.

10. Use of the HDAC binding fragment comprising SEQ ID NO:3 in the manufacture of a composition for use as a medicine in the treatment of proliferative conditions, such as cancer and psoriasis, including but not limited to; lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumours of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumours of mesenchymal origin (e.g. fibrosarcomas and rhabdomyosarcomas), melanomas, teratocarcinomas, neuroblastomas, gliomas, benign tumour of the skin (e.g. keratoacanthomas), breast carcinoma (e.g. advanced breast cancer), kidney carcinoma, ovary carcinoma, bladder carcinoma and epidermal carcinoma.

## Patentansprüche

1. Verfahren für das strukturbasierte oder rationale Konstruieren eines Agonisten oder Antagonisten von HDAC, bei dem die Wechselwirkung eines Adaptor-Proteins, das PH-Domäne, PTB-Domäne und Leucin-Zipper-Motiv enthält (APPL), oder eines mit APPL verwandten Proteins mit einem Histon-Deacetylase (HDAC)-Enzym genutzt wird, wobei das Verfahren die folgenden Schritte umfasst:
a) Sondieren der Struktur der Ligandenbindungsstelle auf HDAC mit APPL oder mit APPL verwandten Proteinen;
b) Identifizieren kontaktierender Atome in der Ligandenbindungsstelle des HDAC-Enzyms, die mit dem APPL-Liganden während der Bindung wechselwirken;
c) Konstruieren von Testverbindungen, die mit den unter (b) identifizierten Atomen wechselwirken, so dass die Aktivität des HDAC-Enzyms moduliert wird; und
d) Inkontaktbringen der konstruierten Testverbindung mit HDAC oder einem funktionsfähigen Fragment davon, um die Fähigkeit der Verbindung, die HDAC-Aktivität zu modulieren, zu messen,
wobei es sich bei dem HDAC-Enzym um ein isoliertes Polypeptid handelt, das die Aminosäuresequenz SEQ ID NO: 5, SEQ ID NO: 6 oder SEQ ID NO: 7 umfasst, und wobei es sich bei dem APPL bzw. mit APPL verwandten Protein um ein isoliertes Polypeptid handelt, das Aminosäuresequenz SEQ ID NO: 2 oder SEQ ID NO: 3 umfasst.

2. Isoliertes und aufgereinigtes APPL bindendes Fragment mit der Aminosäuresequenz SEQ ID NO: 7.

3. Isoliertes und aufgereinigtes Nukleinsäuremolekül, codierend ein von Nukleinsäuren 235 bis 336 unter SEQ ID NO: 4 codiertes APPL bindendes Fragment.

4. Verfahren zur Identifizierung und Gewinnung einer Testverbindung mit der Fähigkeit zur Bindung eines HDAC-Enzyms, umfassend:
a) Inkubieren einer ein HDAC-Enzym oder ein Fragment davon enthaltenden Quelle mit
i) APPL oder mit APPL verwandten Proteinen,
ii) der Testverbindung; und
b) Messen des Effekts der Testverbindung auf die an das Enzym gebundene Menge an APPL bzw. mit APPL verwandten Proteinen,
wobei es sich bei dem HDAC-Enzym oder einem Fragment davon um ein isoliertes Polypeptid handelt, das die Aminosäuresequenz SEQ ID NO: 5, SEQ ID NO: 6 oder SEQ ID NO: 7 umfasst, und wobei es sich bei dem APPL bzw. mit APPL verwandten Protein um ein isoliertes Polypeptid handelt, das Aminosäuresequenz SEQ ID NO: 2 oder SEQ ID NO: 3 umfasst.

5. Verfahren nach Anspruch 4, wobei APPL bzw. das mit APPL verwandte Protein markiert sind und wobei die Markierung zum Messen des Effekts der Testverbindung auf die an das Enzym gebundene Menge an APPL-Proteinen verwendet wird.

6. Verfahren zur Identifizierung und Gewinnung einer Testverbindung mit der Fähigkeit zur Modulation der Aktivität der HDAC, umfassend:
a) Inkubieren einer HDAC oder funktionsfähiger Fragmente davon mit der Testverbindung;
b) Messen des Effekts der Testverbindung auf die Aktivität des HDAC-Enzyms; und
c) Vergleichen dieses Effekts mit der Aktivität des HDAC-Enzyms nach Bindung des APPL-Liganden oder von mit APPL verwandten Proteinen,
wobei es sich bei dem HDAC-Enzym bzw. funktionsfähigen Fragment davon um ein isoliertes Polypeptid handelt, das die Aminosäuresequenz SEQ ID NO: 5, SEQ ID NO: 6 oder SEQ ID NO: 7 umfasst, und wobei es sich bei dem APPL-Liganden bzw. mit APPL verwandten Protein um ein isoliertes Polypeptid handelt, das Aminosäuresequenz SEQ ID NO: 2 oder SEQ ID NO: 3 umfasst.

7. Verfahren nach Anspruch 6, wobei der Effekt der Testverbindung auf das HDAC-Enzym unter Verwendung eines radioaktiv markierten oder fluoreszenzmarkierten acetylierten Histons als Substrat und Messen der Freisetzung der markierten Acetylgruppe beurteilt wird.

8. Verfahren nach Anspruch 7, wobei es sich bei dem Substrat um radioaktiv markiertes acetyliertes Histon-H4-Peptid handelt.

9. APPL bindendes Fragment mit Aminosäuresequenz SEQ ID NO: 7 zur Verwendung als Medizin.

10. Verwendung des HDAC bindenden Fragments, umfassend SEQ ID NO: 3, bei der Herstellung einer Zusammensetzung zur Verwendung als Medizin bei der Behandlung proliferativer Leiden wie Krebs und Psoriasis, einschließlich, ohne jedoch darauf beschränkt zu sein, Lungenkrebs (z.B. Adenokarzinom und einschließlich nichtkleinzelligem Lungenkrebs), Krebserkrankungen des Pankreas (z.B. Pankreaskarzinom wie z.B. exokrinem Pankreaskarzinom), Krebserkrankungen des Dickdarms (z.B. Kolorektalkarzinomen, wie z.B. Kolon-Adenokarzinom und Kolon-Adenom), Prostatakrebs, einschließlich der fortgeschrittenen Krankheit, hämatopoetischen Tumoren der Lymphoidlinie (z.B. akuter lymphatischer Leukämie, B-Zellen-Lymphom, Burkitt-Lymphom), myeloischer Leukämien (z.B. akuter myeloischer Leukämie (AML)), follikulärem Schilddrüsenkrebs, myelodysplastischem Syndrom (MDS), Tumoren mesenchymalen Ursprungs (z.B. Fibrosarkomen und Rhabdomyosarkomen), Melanomen, Teratokarzinomen, Neuroblastomen, Gliomen, gutartigem Hauttumor (z.B. Keratoakanthomen), Brustkarzinom (z.B. fortgeschrittenem Brustkrebs), Nierenkarzinom, Ovarialkarzinom, Blasenkarzinom und Karzinom der Epidermis.

## Revendications

1. Procédé pour la conception structurelle ou rationnelle d'un agoniste ou d'un antagoniste de la HDAC, qui utilise l'interaction d'une protéine adaptatrice contenant un domaine PH, un domaine PTB et un motif de glissière à leucine (APPL) ou une protéine associée à l'APPL avec une enzyme histone désacétylase (HDAC), ledit procédé comprenant les étapes :
a) sondage de la structure du site de liaison au ligand sur la HDAC avec des protéines APPL ou associées à l'APPL ;
b) identification des atomes en contact dans le site de liaison au ligand de l'enzyme HDAC qui interagissent avec le ligand APPL pendant la liaison ;
c) conception de composés d'essai qui interagissent avec les atomes identifiés dans (b) pour moduler l'activité de l'enzyme HDAC ; et
d) mise en contact dudit composé d'essai conçu avec la HDAC ou un fragment fonctionnel de celle-ci, pour mesurer la capacité dudit composé à moduler l'activité de HDAC,
dans lequel l'enzyme HDAC est un polypeptide isolé comprenant la séquence d'acides aminés SEQ ID NO:5, SEQ ID NO:6 ou SEQ ID NO:7, et dans lequel la protéine APPL ou associée à l'APPL est un polypeptide isolé comprenant la séquence d'acides aminés SEQ ID NO:2 ou SEQ ID NO:3.

2. Fragment de liaison à l'APPL, isolé et purifié, ayant la séquence d'acides aminés SEQ ID NO:7.

3. Molécule d'acide nucléique isolée et purifiée codant pour un fragment de liaison à l'APPL, codée par les acides nucléiques 235 à 336 de SEQ ID NO:4.

4. Procédé pour identifier et obtenir un composé d'essai capable de se lier à une enzyme HDAC, comprenant :
a) l'incubation d'une source contenant une enzyme HDAC ou un fragment de celle-ci, avec i) des protéines APPL ou associées à l'APPL, ii)ledit composé d'essai ; et
b) la mesure de l'effet du composé d'essai sur la quantité de protéines APPL ou associées à l'APPL liées à l'enzyme,
dans lequel l'enzyme HDAC ou un fragment de celle-ci est un polypeptide isolé comprenant la séquence d'acides aminés SEQ ID NO:5, SEQ ID NO:6 ou SEQ ID NO:7, et dans lequel la protéine APPL ou associée à l'APPL est un polypeptide isolé comprenant la séquence d'acides aminés SEQ ID NO:2 ou SEQ ID NO:3.

5. Procédé selon la revendication 4, dans lequel la protéine APPL ou associée à l'APPL est marquée, et dans lequel ledit marqueur est utilisé pour mesurer l'effet du composé d'essai sur la quantité de protéines APPL liées à l'enzyme.

6. Procédé d'identification et d'obtention d'un composé d'essai capable de moduler l'activité des HDAC, comprenant :
a) l'incubation d'une HDAC ou de fragments fonctionnels de celle-ci, avec ledit composé d'essai ;
b) mesure de l'effet de ce composé d'essai sur l'activité de l'enzyme HDAC ; et
c) comparaison de cet effet avec l'activité de l'enzyme HDAC après liaison du ligand APPL ou des protéines associées à l'APPL,
dans lequel l'enzyme HDAC, ou un fragment fonctionnel de celle-ci, est un polypeptide isolé comprenant la séquence d'acides aminés SEQ ID NO:5, SEQ ID NO:6 ou SEQ ID NO:7, et dans lequel le ligand APPL ou la protéine associée à l'APPL est un polypeptide isolé comprenant la séquence d'acides aminés SEQ ID NO:2 ou SEQ ID NO:3.

7. Procédé selon la revendication 6, dans lequel l'effet du composé d'essai sur l'enzyme HDAC est évalué par utilisation, en tant que substrat, d'une histone acétylée radiomarquée ou marquée par fluorescence, et mesure de la libération du groupe acétyl marqué.

8. Procédé selon la revendication 7, dans lequel le substrat est un peptide d'histone H4 acétylée radiomarquée.

9. Fragment de liaison à l'APPL ayant la séquence d'acides aminés SEQ ID NO:7, pour une utilisation en tant que médicament.

10. Utilisation du fragment de liaison à la HDAC comprenant SEQ ID NO:3 pour la fabrication d'une composition destinée à être utilisée en tant que médicament dans le traitement d'états pathologiques prolifératifs tels que le cancer et le psoriasis, y compris, mais sans y être limité, le cancer du poumon (p.ex. un adénocarcinome, et comprenant un cancer du poumon non à petites cellules), le cancer pancréatique (p.ex. le carcinome pancréatique tel que par exemple le carcinome pancréatique exocrine), les cancers du côlon (p.ex. les carcinomes colorectaux, tels que par exemple l'adénocarcinome du côlon et l'adénome du côlon), le cancer de la prostate, y compris la maladie avancée, les tumeurs hématopoïétiques de la lignée lymphoïde (p.ex. la leucémie lymphocytaire aiguë, le lymphome à cellules B, le lymphome de Burkitt), les leucémies myéloïdes (p.ex. la leucémie myélogène aiguë (AML)), le cancer folliculaire de la thyroïde, le syndrome myélodysplasique (MDS), les tumeurs d'origine mésenchymateuse (p.ex. les fibrosarcomes et les rhabdomyosarcomes), les mélanomes, les tératocarcinomes, les neuroblastomes, les gliomes, une tumeur bénigne de la peau (p.ex. les kératoacanthomes), le cancer du sein (p.ex. le cancer du sein avancé), le cancer du rein, le cancer des ovaires, le cancer de la vessie et le cancer de l'épiderme.
